# EUROPÄISCHE PATENTSCHRIFT

(11) **EP 2 270 029 B1**
(45) Veröffentlichungstag und Bekanntmachung des Hinweises auf die Patenterteilung: **19.11.2014**
(21) Anmeldenummer: 10168383.7
(22) Anmeldetag: 03.07.2010
(51) Int. Cl.: C07K 14/195, C07K 14/32, C12N 15/62

(54) **E. coli Sekretionssystem auf der Basis von S-Layer-Proteinen**
E.coli secretion system on the basis of S-layer proteins
Système de sécrétion d'E. coli à base de protéines à couche S

(30) Priorität: 03.07.2009 DE 102009032646
(43) Veröffentlichungstag der Anmeldung: 05.01.2011
(73) Patentinhaber: Helmholtz-Zentrum Dresden - Rossendorf e.V., 01328 Dresden (DE)
(72) Erfinder: Pollmann, Katrin, 01324, Dresden (DE); Lederer, Franziska, 01920, Oßling OT Weißig (DE); Raff, Johannes, Dr., 01324, Dresden (DE)
(74) Vertreter: Kailuweit & Uhlemann Patentanwälte Partnerschaft mbB

(56) Entgegenhaltungen:
- WO-A1-00/49163
- WO-A1-97/28263
- US-A1- 2002 048 816
- DATABASE BIOSIS [Online] BIOSCIENCES INFORMATION SERVICE, PHILADELPHIA, PA, US; September 2005 (2005-09), POLLMANN KATRIN ET AL: "Novel surface layer protein genes in Bacillus sphaericus associated with unusual insertion elements" XP009138823 Database accession no. PREV200510342926
- DATABASE BIOSIS [Online] BIOSCIENCES INFORMATION SERVICE, PHILADELPHIA, PA, US; 11. April 2003 (2003-04-11), IRIHIMOVITCH VERED ET AL: "Post-translational secretion of fusion proteins in the halophilic Archaea Haloferax volcanii." XP009138834 Database accession no. PREV200300253966
- BINGLE W H ET AL: "LINKER MUTAGENESIS OF THE CAULOBACTER CRESCENTUS S-LAYER PROTEIN: TOWARD A DEFINITION OF AN N-TERMINAL ANCHORING REGION AND A C-TERMINAL SECRETION SIGNAL AND THE POTENTIAL FOR HETEROLOGOUS PROTEIN SECRETION" JOURNAL OF BACTERIOLOGY, AMERICAN SOCIETY FOR MICROBIOLOGY, WASHINGTON, DC; US, Bd. 179, Nr. 3, 1. Februar 1997 (1997-02-01), Seiten 601-611, XP002034945 ISSN: 0021-9193
- SAVIJOKI K ET AL: "High level heterologous protein production in Lactococcus and Lactobacillus using a new secretion system based on the Lactobacillus brevis S-layer signals" GENE, ELSEVIER, AMSTERDAM, NL LNKD- DOI:10.1016/S0378-1119(96)00717-2, Bd. 186, Nr. 2, 28. Februar 1997 (1997-02-28), Seiten 255-262, XP004093308 ISSN: 0378-1119
- BOWDITCH R D ET AL: "CLONING AND SEQUENCING OF THE GENE ENCODING A 125-KILODALTON SURFACE-LAYER PROTEIN FROM BACILLUS SPHAERICUS 2362 AND OF A RELATED CRYPTIC GENE", JOURNAL OF BACTERIOLOGY, AMERICAN SOCIETY FOR MICROBIOLOGY, WASHINGTON, DC; US, vol. 171, no. 8, 1 August 1989 (1989-08-01), pages 4178-4188, XP000674383, ISSN: 0021-9193

### SEQUENCE LISTING

<110> Forschungszentrum Dresden-Rossendorf e. v.
<120> E. coli-Sekretionssystem auf der Basis von S-Layer-Proteinen
<130> P0904EP
<150> DE 10 2009 032 646.4
   <151> 2009-07-03
<160> 12
<170> PatentIn version 3.5
<210> 1
   <211> 3684
   <212> DNA
   <213> Lysinibacillus sphaericus
<220>
   <221> CDS
   <222> (1)..(3684)
<220>
   <221> misc_feature
   <222> (411)..(411)
   <223> n is a, c, g, or t
<400> 1
   atg gct aac caa cca aag aaa tac aaa aaa ttc gta gcg acg gca gca      48
   Met Ala Asn Gln Pro Lys Lys Tyr Lys Lys Phe Val Ala Thr Ala Ala
   1               5                   10                  15
   acg gca aca tta gta gca tct gct atc gta cca gtg gtt tct gcc gca      96
   Thr Ala Thr Leu Val Ala Ser Ala Ile Val Pro Val Val Ser Ala Ala
               20                  25                  30
   gga ttt tca gat gta gca ggt aac gac cac gaa gta gca atc aac gca     144
   Gly Phe Ser Asp Val Ala Gly Asn Asp His Glu Val Ala Ile Asn Ala
           35                  40                  45
   ctt gct gat gca ggt atc atc aat gga tac gct gac ggc aca ttc aaa     192
   Leu Ala Asp Ala Gly Ile Ile Asn Gly Tyr Ala Asp Gly Thr Phe Lys
       50                  55                  60
   cca aac caa aca att aac cgc ggt caa gtg gtt aaa tta tta ggt cgt     240
   Pro Asn Gln Thr Ile Asn Arg Gly Gln Val Val Lys Leu Leu Gly Arg
   65                  70                  75                  80
   tat tta gaa gca caa ggc caa gaa att cca gct gac tgg aat tca aaa     288
   Tyr Leu Glu Ala Gln Gly Gln Glu Ile Pro Ala Asp Trp Asn Ser Lys
                   85                  90                  95
   caa cgc ttc aac gat cta cca gta aca gct gaa gca gaa tta gta aaa     336
   Gln Arg Phe Asn Asp Leu Pro Val Thr Ala Glu Ala Glu Leu Val Lys
               100                 105                 110
   tat gct gca cta gca aaa gat gca ggc gta ttc aac ggt tca aac ggc     384
   Tyr Ala Ala Leu Ala Lys Asp Ala Gly Val Phe Asn Gly Ser Asn Gly
           115                 120                 125
   aac tta aac gct tca caa aca atg can cgt caa caa atg gca gta gtt     432
   Asn Leu Asn Ala Ser Gln Thr Met Xaa Arg Gln Gln Met Ala Val Val
       130                 135                 140
   tta gta cgt gct att aaa gaa atc gct ggc gta gac tta gta gct gag     480
   Leu Val Arg Ala Ile Lys Glu Ile Ala Gly Val Asp Leu Val Ala Glu
   145                 150                 155                 160
   tac aaa aaa gcg aat ttc gta aca gaa att ggt gac tta gac aaa gct      528
   Tyr Lys Lys Ala Asn Phe Val Thr Glu Ile Gly Asp Leu Asp Lys Ala
                   165                 170                 175
   tac tct gca gaa caa cgc aca gcg atc gtt gct tta gaa tat gcg ggt      576
   Tyr Ser Ala Glu Gln Arg Thr Ala Ile Val Ala Leu Glu Tyr Ala Gly
               180                 185                 190
   att aca aat gtt gct cac ttc aac cca ggt aac agc gta aca cgc gga      624
   Ile Thr Asn Val Ala His Phe Asn Pro Gly Asn Ser Val Thr Arg Gly
           195                 200                 205
   caa ttc gct tca ttc tta tac cgc aca atc gaa aac gtg gta aac gct      672
   Gln Phe Ala Ser Phe Leu Tyr Arg Thr Ile Glu Asn Val Val Asn Ala
       210                 215                 220
   cct gaa gct ggc gct gcg gca gtg aaa gct gta aac aac aca act gtt      720
   Pro Glu Ala Gly Ala Ala Ala Val Lys Ala Val Asn Asn Thr Thr Val
   225                 230                 235                 240
   gaa gta aca ttc gac gaa gaa gta gac aac gta caa gct ctt aac ttc      768
   Glu Val Thr Phe Asp Glu Glu Val Asp Asn Val Gln Ala Leu Asn Phe
                   245                 250                 255
   tta atc tct gat tta gaa gtg aaa aac gca gct gta aaa caa act aac      816
   Leu Ile Ser Asp Leu Glu Val Lys Asn Ala Ala Val Lys Gln Thr Asn
               260                 265                 270
   aaa aaa gtt gtt gtt tta act act gca cct caa act gct gac aaa gag      864
   Lys Lys Val Val Val Leu Thr Thr Ala Pro Gln Thr Ala Asp Lys Glu
           275                 280                 285
   tac act gta tct ctt ggc gaa gaa aaa att ggt act ttc aaa ggt atc      912
   Tyr Thr Val Ser Leu Gly Glu Glu Lys Ile Gly Thr Phe Lys Gly Ile
       290                 295                 300
   gca gct gta gtt cct act aaa gta gat tta gta gaa aaa tca gtt caa      960
   Ala Ala Val Val Pro Thr Lys Val Asp Leu Val Glu Lys Ser Val Gln
   305                 310                 315                 320
   ggt aaa ctt ggc caa caa gta aca ctg aaa gca caa gta aca gtt gct     1008
   Gly Lys Leu Gly Gln Gln Val Thr Leu Lys Ala Gln Val Thr Val Ala
                   325                 330                 335
   gaa ggt caa aca aaa gct ggt att cca gta act ttc ttc atc cca ggt     1056
   Glu Gly Gln Thr Lys Ala Gly Ile Pro Val Thr Phe Phe Ile Pro Gly
               340                 345                 350
   tct gct aat ggt gtg aaa tct cca gta aca gtt gaa gct gta aca gat     1104
   Ser Ala Asn Gly Val Lys Ser Pro Val Thr Val Glu Ala Val Thr Asp
           355                 360                 365
   gaa aac ggt gtt gct tct tat act tac act cgt tac gct gca aca aac     1152
   Glu Asn Gly Val Ala Ser Tyr Thr Tyr Thr Arg Tyr Ala Ala Thr Asn
       370                 375                 380
   gac aca gta act gta tac gcg aac ggt gac cgt tct aaa ttc tca act     1200
   Asp Thr Val Thr Val Tyr Ala Asn Gly Asp Arg Ser Lys Phe Ser Thr
   385                 390                 395                 400
   ggt tat gtc ttc tgg gct gtt gat aca act cta gaa atc act gaa gta     1248
   Gly Tyr Val Phe Trp Ala Val Asp Thr Thr Leu Glu Ile Thr Glu Val
                   405                 410                 415
   act act ggc gat att atc aac aac ggt gct aac aaa act tat aaa gtt     1296
   Thr Thr Gly Asp Ile Ile Asn Asn Gly Ala Asn Lys Thr Tyr Lys Val
               420                 425                 430
   gca tat aaa gac cca gaa act ggt aaa cca gta tca ggt aaa gta ctt       1344
   Ala Tyr Lys Asp Pro Glu Thr Gly Lys Pro Val Ser Gly Lys Val Leu
           435                 440                 445
   aac gtt tct gta aaa gaa aac att gac gta act gtt gat aaa tta caa       1392
   Asn Val Ser Val Lys Glu Asn Ile Asp Val Thr Val Asp Lys Leu Gln
       450                 455                 460
   aac gtt aca att aat ggt att aaa gta gct caa act agc act tca aat       1440
   Asn Val Thr Ile Asn Gly Ile Lys Val Ala Gln Thr Ser Thr Ser Asn
   465                 470                 475                 480
   act aca gct gca caa gtt aca act gac tct aaa ggt gaa gca aca ttc       1488
   Thr Thr Ala Ala Gln Val Thr Thr Asp Ser Lys Gly Glu Ala Thr Phe
                   485                 490                 495
   act att act ggc gca aat gct gaa gta act cca gtt gta ttt gct ggc       1536
   Thr Ile Thr Gly Ala Asn Ala Glu Val Thr Pro Val Val Phe Ala Gly
               500                 505                 510
   gaa gca gtt act ggt caa aca tta cct tct cag aaa tat aat gct gat       1584
   Glu Ala Val Thr Gly Gln Thr Leu Pro Ser Gln Lys Tyr Asn Ala Asp
           515                 520                 525
   gca tta caa aca act gct aaa aag gta aaa ttc ggt gcg ctt caa gct       1632
   Ala Leu Gln Thr Thr Ala Lys Lys Val Lys Phe Gly Ala Leu Gln Ala
       530                 535                 540
   gac tat att ctt gaa gta act cgt gac ggt ggc gaa aca gct gct act       1680
   Asp Tyr Ile Leu Glu Val Thr Arg Asp Gly Gly Glu Thr Ala Ala Thr
   545                 550                 555                 560
   gga tat gat aac gga cgt aaa tat aaa ctt gtc gtt aaa gat aaa aac       1728
   Gly Tyr Asp Asn Gly Arg Lys Tyr Lys Leu Val Val Lys Asp Lys Asn
                   565                 570                 575
   ggt aaa tta gca gca aat gaa aca gta aat gtt gca ttt aac gaa gat       1776
   Gly Lys Leu Ala Ala Asn Glu Thr Val Asn Val Ala Phe Asn Glu Asp
               580                 585                 590
   atc gat ggc gtt atc tct aca act act gat gct aaa ttt gtt aaa tta       1824
   Ile Asp Gly Val Ile Ser Thr Thr Thr Asp Ala Lys Phe Val Lys Leu
           595                 600                 605
   gat aac gaa gac aat caa gtt ggt tgg gat act act gta gat aaa gat       1872
   Asp Asn Glu Asp Asn Gln Val Gly Trp Asp Thr Thr Val Asp Lys Asp
       610                 615                 620
   gct aag aaa atc act gtt aaa aca gat tca aaa ggt gaa gct agc ttc       1920
   Ala Lys Lys Ile Thr Val Lys Thr Asp Ser Lys Gly Glu Ala Ser Phe
   625                 630                 635                 640
   att atc ggt aat act aaa gaa aac aca tat gst aca cca atc gct tgg       1968
   Ile Ile Gly Asn Thr Lys Glu Asn Thr Tyr Xaa Thr Pro Ile Ala Trp
                   645                 650                 655
   att gat gtt aac tat caa agt ggt aaa gtt gga aca tta gac caa gga       2016
   Ile Asp Val Asn Tyr Gln Ser Gly Lys Val Gly Thr Leu Asp Gln Gly
               660                 665                 670
   gag cca agt gca act gct cca atc tct tac ttc caa tct cca act att       2064
   Glu Pro Ser Ala Thr Ala Pro Ile Ser Tyr Phe Gln Ser Pro Thr Ile
           675                 680                 685
   gat ggt tct aaa tta gtt gca gaa aat aaa gat ggt aaa aaa cct tct       2112
   Asp Gly Ser Lys Leu Val Ala Glu Asn Lys Asp Gly Lys Lys Pro Ser
       690                 695                 700
   gat ttt gaa gat aaa gat gct atc ttt aaa gta gta ctt gta aac caa      2160
   Asp Phe Glu Asp Lys Asp Ala Ile Phe Lys Val Val Leu Val Asn Gln
   705                 710                 715                 720
   agt ggt aaa gaa atg aaa aac cac aat tac aaa aca gaa aaa gtt tct      2208
   Ser Gly Lys Glu Met Lys Asn His Asn Tyr Lys Thr Glu Lys Val Ser
                   725                 730                 735
   tat acg gtg aat aac act ggt tct aat gag gct gtt gta gag tac aca      2256
   Tyr Thr Val Asn Asn Thr Gly Ser Asn Glu Ala Val Val Glu Tyr Thr
               740                 745                 750
   tat aac ggt aaa gtt gag tca gaa aca ctt gct cca aat cgt gct act      2304
   Tyr Asn Gly Lys Val Glu Ser Glu Thr Leu Ala Pro Asn Arg Ala Thr
           755                 760                 765
   tct gta gtt gct gat aat ggt aca att aca gtt aaa cca aat ggt aaa      2352
   Ser Val Val Ala Asp Asn Gly Thr Ile Thr Val Lys Pro Asn Gly Lys
       770                 775                 780
   act act tct gta aaa gtt cta gca act ggt gtt gca aaa gaa gat aaa      2400
   Thr Thr Ser Val Lys Val Leu Ala Thr Gly Val Ala Lys Glu Asp Lys
   785                 790                 795                 800
   act aat ggt aaa gaa tat gca ttc act gct aaa gaa gca act gca aaa      2448
   Thr Asn Gly Lys Glu Tyr Ala Phe Thr Ala Lys Glu Ala Thr Ala Lys
                   805                 810                 815
   ttc aca gct act aaa gaa att tca aac cca tat act ggt gcg att aaa      2496
   Phe Thr Ala Thr Lys Glu Ile Ser Asn Pro Tyr Thr Gly Ala Ile Lys
               820                 825                 830
   tac tac gat act gat aag aaa act atc act ttc gat ggt aaa gac gca      2544
   Tyr Tyr Asp Thr Asp Lys Lys Thr Ile Thr Phe Asp Gly Lys Asp Ala
           835                 840                 845
   atc aaa tat gct ggt gaa tct ggt aag aaa tat aaa tac tac agc ggt      2592
   Ile Lys Tyr Ala Gly Glu Ser Gly Lys Lys Tyr Lys Tyr Tyr Ser Gly
       850                 855                 860
   aac att gaa gtt gca aca gaa aaa gca ttt att gat tta tta gct aat      2640
   Asn Ile Glu Val Ala Thr Glu Lys Ala Phe Ile Asp Leu Leu Ala Asn
   865                 870                 875                 880
   gct tct gga aaa gta act gtt act tat aaa gtt gaa gat gat act aaa      2688
   Ala Ser Gly Lys Val Thr Val Thr Tyr Lys Val Glu Asp Asp Thr Lys
                   885                 890                 895
   tca ttc cat att att aac gtt gga act gat gta gct aat aaa cct gta      2736
   Ser Phe His Ile Ile Asn Val Gly Thr Asp Val Ala Asn Lys Pro Val
               900                 905                 910
   gaa aag aaa gat gat aca aaa cct aca aac aat gca cct gtt gct aaa      2784
   Glu Lys Lys Asp Asp Thr Lys Pro Thr Asn Asn Ala Pro Val Ala Lys
           915                 920                 925
   caa gtt gct gat caa gtt att gcg tct aat gga agt aaa tta aca ttt      2832
   Gln Val Ala Asp Gln Val Ile Ala Ser Asn Gly Ser Lys Leu Thr Phe
       930                 935                 940
   aca gct aat gat gta gca tca gat gct gat aaa gat act tta aaa att      2880
   Thr Ala Asn Asp Val Ala Ser Asp Ala Asp Lys Asp Thr Leu Lys Ile
   945                 950                 955                 960
   gtt act gca tat act aca aat tct aca gta gca act gca act act gat      2928
   Val Thr Ala Tyr Thr Thr Asn Ser Thr Val Ala Thr Ala Thr Thr Asp
                   965                 970                 975
   gct aac caa tta ggt ttc tct gta gaa cca aaa ggt gaa ggt tca aca      2976
   Ala Asn Gln Leu Gly Phe Ser Val Glu Pro Lys Gly Glu Gly Ser Thr
           980                     985                     990
   act gta act gtt gta gtt act gat ggt aaa gat aat att aat gta agc     3024
   Thr Val Thr Val Val Val Thr Asp Gly Lys Asp Asn Ile Asn Val Ser
       995                 1000                1005
   ttc aag gtt aat gta tct act gct agc tat act tct gta gta aac         3069
   Phe Lys Val Asn Val Ser Thr Ala Ser Tyr Thr Ser Val Val Asn
       1010                1015                1020
   tct ggt aac cca gtt aaa gat ttt gct cct gca gtt cct aca gca         3114
   Ser Gly Asn Pro Val Lys Asp Phe Ala Pro Ala Val Pro Thr Ala
       1025                1030                1035
   gca aca gca aaa cta cca gaa ttc act tct gta gca aaa gct ggt         3159
   Ala Thr Ala Lys Leu Pro Glu Phe Thr Ser Val Ala Lys Ala Gly
       1040                1045                1050
   act att aaa gta caa gga ttt gat att cct tta aca ctt ggt tct         3204
   Thr Ile Lys Val Gln Gly Phe Asp Ile Pro Leu Thr Leu Gly Ser
       1055                1060                1065
   act caa gca caa gtg cta gag aca ctt aat aat ttt att act gac         3249
   Thr Gln Ala Gln Val Leu Glu Thr Leu Asn Asn Phe Ile Thr Asp
       1070                1075                1080
   tat aaa att aat gca agt gct aaa ctt gtt gat aaa gcg att gta         3294
   Tyr Lys Ile Asn Ala Ser Ala Lys Leu Val Asp Lys Ala Ile Val
       1085                1090                1095
   att agc tct act gaa aca ggt aac gct gct act cta aca gtt gaa         3339
   Ile Ser Ser Thr Glu Thr Gly Asn Ala Ala Thr Leu Thr Val Glu
       1100                1105                1110
   ggt gat agt aca tta gaa ctt gta gct ggt act gta tta ggt act         3384
   Gly Asp Ser Thr Leu Glu Leu Val Ala Gly Thr Val Leu Gly Thr
       1115                1120                1125
   gtt tca act aca cct ggt tca gtt ggt act gct act cct gct aaa         3429
   Val Ser Thr Thr Pro Gly Ser Val Gly Thr Ala Thr Pro Ala Lys
       1130                1135                1140
   tac gct gta aaa gta tta aac ggt gta agt aac gga aca aaa gtt         3474
   Tyr Ala Val Lys Val Leu Asn Gly Val Ser Asn Gly Thr Lys Val
       1145                1150                1155
   gat ttc aaa ttt aca att ggt gca aaa aca gta aca gta act gtt         3519
   Asp Phe Lys Phe Thr Ile Gly Ala Lys Thr Val Thr Val Thr Val
       1160                1165                1170
   gat gca act gct ggt gca aaa gca gta tct gac gtt gta gat act         3564
   Asp Ala Thr Ala Gly Ala Lys Ala Val Ser Asp Val Val Asp Thr
       1175                1180                1185
   tta gct gct gca aca tta gat gga tac tca atc act aaa aat gga         3609
   Leu Ala Ala Ala Thr Leu Asp Gly Tyr Ser Ile Thr Lys Asn Gly
       1190                1195                1200
   gat gtt att gag tta act caa aca act cca gca aca act act gat         3654
   Asp Val Ile Glu Leu Thr Gln Thr Thr Pro Ala Thr Thr Thr Asp
       1205                1210                1215
   aaa tta gtt gtt gaa act aaa aaa gct aac                             3684
   Lys Leu Val Val Glu Thr Lys Lys Ala Asn
       1220                1225
<210> 2
   <211> 1228
   <212> PRT
   <213> Lysinibacillus sphaericus
<220>
   <221> misc_feature
   <222> (137)..(137)
   <223> The 'Xaa' at location 137 stands for Gln, or His.
<220>
   <221> misc_feature
   <222> (651)..(651)
   <223> The 'Xaa' at location 651 stands for Gly, or Ala.
<400> 2
   Met Ala Asn Gln Pro Lys Lys Tyr Lys Lys Phe Val Ala Thr Ala Ala
   1               5                   10                  15
   Thr Ala Thr Leu Val Ala Ser Ala Ile Val Pro Val Val Ser Ala Ala
               20                  25                  30
   Gly Phe Ser Asp Val Ala Gly Asn Asp His Glu Val Ala Ile Asn Ala
           35                  40                  45
   Leu Ala Asp Ala Gly Ile Ile Asn Gly Tyr Ala Asp Gly Thr Phe Lys
       50                  55                  60
   Pro Asn Gln Thr Ile Asn Arg Gly Gln Val Val Lys Leu Leu Gly Arg
   65                  70                  75                  80
   Tyr Leu Glu Ala Gln Gly Gln Glu Ile Pro Ala Asp Trp Asn Ser Lys
                   85                  90                  95
   Gln Arg Phe Asn Asp Leu Pro Val Thr Ala Glu Ala Glu Leu Val Lys
               100                 105                 110
   Tyr Ala Ala Leu Ala Lys Asp Ala Gly Val Phe Asn Gly Ser Asn Gly
           115                 120                 125
   Asn Leu Asn Ala Ser Gln Thr Met Xaa Arg Gln Gln Met Ala Val Val
       130                 135                 140
   Leu Val Arg Ala Ile Lys Glu Ile Ala Gly Val Asp Leu Val Ala Glu
   145                 150                 155                 160
   Tyr Lys Lys Ala Asn Phe Val Thr Glu Ile Gly Asp Leu Asp Lys Ala
                   165                 170                 175
   Tyr Ser Ala Glu Gln Arg Thr Ala Ile Val Ala Leu Glu Tyr Ala Gly
               180                 185                 190
   Ile Thr Asn Val Ala His Phe Asn Pro Gly Asn Ser Val Thr Arg Gly
           195                 200                 205
   Gln Phe Ala Ser Phe Leu Tyr Arg Thr Ile Glu Asn Val Val Asn Ala
   210 215 220
   Pro Glu Ala Gly Ala Ala Ala Val Lys Ala Val Asn Asn Thr Thr Val
   225 230 235 240
   Glu Val Thr Phe Asp Glu Glu Val Asp Asn Val Gln Ala Leu Asn Phe
   245 250 255
   Leu Ile Ser Asp Leu Glu Val Lys Asn Ala Ala Val Lys Gln Thr Asn
   260 265 270
   Lys Lys Val Val Val Leu Thr Thr Ala Pro Gln Thr Ala Asp Lys Glu
   275 280 285
   Tyr Thr Val Ser Leu Gly Glu Glu Lys Ile Gly Thr Phe Lys Gly Ile
   290 295 300
   Ala Ala Val Val Pro Thr Lys Val Asp Leu Val Glu Lys Ser Val Gln
   305 310 315 320
   Gly Lys Leu Gly Gln Gln Val Thr Leu Lys Ala Gln Val Thr Val Ala
   325 330 335
   Glu Gly Gln Thr Lys Ala Gly Ile Pro Val Thr Phe Phe Ile Pro Gly
   340 345 350
   Ser Ala Asn Gly Val Lys Ser Pro Val Thr Val Glu Ala Val Thr Asp
   355 360 365
   Glu Asn Gly Val Ala Ser Tyr Thr Tyr Thr Arg Tyr Ala Ala Thr Asn
   370 375 380
   Asp Thr Val Thr Val Tyr Ala Asn Gly Asp Arg Ser Lys Phe Ser Thr
   385 390 395 400
   Gly Tyr Val Phe Trp Ala Val Asp Thr Thr Leu Glu Ile Thr Glu Val
   405 410 415
   Thr Thr Gly Asp Ile Ile Asn Asn Gly Ala Asn Lys Thr Tyr Lys Val
   420 425 430
   Ala Tyr Lys Asp Pro Glu Thr Gly Lys Pro Val Ser Gly Lys Val Leu
   435 440 445
   Asn Val Ser Val Lys Glu Asn Ile Asp Val Thr Val Asp Lys Leu Gln
   450 455 460
   Asn Val Thr Ile Asn Gly Ile Lys Val Ala Gln Thr Ser Thr Ser Asn
   465 470 475 480
   Thr Thr Ala Ala Gln Val Thr Thr Asp Ser Lys Gly Glu Ala Thr Phe
   485 490 495
   Thr Ile Thr Gly Ala Asn Ala Glu Val Thr Pro Val Val Phe Ala Gly
   500 505 510
   Glu Ala Val Thr Gly Gln Thr Leu Pro Ser Gln Lys Tyr Asn Ala Asp
   515 520 525
   Ala Leu Gln Thr Thr Ala Lys Lys Val Lys Phe Gly Ala Leu Gln Ala
   530 535 540
   Asp Tyr Ile Leu Glu Val Thr Arg Asp Gly Gly Glu Thr Ala Ala Thr
   545 550 555 560
   Gly Tyr Asp Asn Gly Arg Lys Tyr Lys Leu Val Val Lys Asp Lys Asn
   565 570 575
   Gly Lys Leu Ala Ala Asn Glu Thr Val Asn Val Ala Phe Asn Glu Asp
   580 585 590
   Ile Asp Gly Val Ile Ser Thr Thr Thr Asp Ala Lys Phe Val Lys Leu
   595 600 605
   Asp Asn Glu Asp Asn Gln Val Gly Trp Asp Thr Thr Val Asp Lys Asp
   610 615 620
   Ala Lys Lys Ile Thr Val Lys Thr Asp Ser Lys Gly Glu Ala Ser Phe
   625 630 635 640
   Ile Ile Gly Asn Thr Lys Glu Asn Thr Tyr Xaa Thr Pro Ile Ala Trp
   645 650 655
   Ile Asp Val Asn Tyr Gln Ser Gly Lys Val Gly Thr Leu Asp Gln Gly
   660 665 670
   Glu Pro Ser Ala Thr Ala Pro Ile Ser Tyr Phe Gln Ser Pro Thr Ile
   675 680 685
   Asp Gly Ser Lys Leu Val Ala Glu Asn Lys Asp Gly Lys Lys Pro Ser
   690 695 700
   Asp Phe Glu Asp Lys Asp Ala Ile Phe Lys Val Val Leu Val Asn Gln
   705 710 715 720
   Ser Gly Lys Glu Met Lys Asn His Asn Tyr Lys Thr Glu Lys Val Ser
   725 730 735
   Tyr Thr Val Asn Asn Thr Gly Ser Asn Glu Ala Val Val Glu Tyr Thr
   740 745 750
   Tyr Asn Gly Lys Val Glu Ser Glu Thr Leu Ala Pro Asn Arg Ala Thr
   755 760 765
   Ser Val Val Ala Asp Asn Gly Thr Ile Thr Val Lys Pro Asn Gly Lys
   770 775 780
   Thr Thr Ser Val Lys Val Leu Ala Thr Gly Val Ala Lys Glu Asp Lys
   785 790 795 800
   Thr Asn Gly Lys Glu Tyr Ala Phe Thr Ala Lys Glu Ala Thr Ala Lys
   805 810 815
   Phe Thr Ala Thr Lys Glu Ile Ser Asn Pro Tyr Thr Gly Ala Ile Lys
   820 825 830
   Tyr Tyr Asp Thr Asp Lys Lys Thr Ile Thr Phe Asp Gly Lys Asp Ala
   835 840 845
   Ile Lys Tyr Ala Gly Glu Ser Gly Lys Lys Tyr Lys Tyr Tyr Ser Gly
   850 855 860
   Asn Ile Glu Val Ala Thr Glu Lys Ala Phe Ile Asp Leu Leu Ala Asn
   865 870 875 880
   Ala Ser Gly Lys Val Thr Val Thr Tyr Lys Val Glu Asp Asp Thr Lys
   885 890 895
   Ser Phe His Ile Ile Asn Val Gly Thr Asp Val Ala Asn Lys Pro Val
   900 905 910
   Glu Lys Lys Asp Asp Thr Lys Pro Thr Asn Asn Ala Pro Val Ala Lys
   915 920 925
   Gln Val Ala Asp Gln Val Ile Ala Ser Asn Gly Ser Lys Leu Thr Phe
   930 935 940
   Thr Ala Asn Asp Val Ala Ser Asp Ala Asp Lys Asp Thr Leu Lys Ile
   945 950 955 960
   Val Thr Ala Tyr Thr Thr Asn Ser Thr Val Ala Thr Ala Thr Thr Asp
   965 970 975
   Ala Asn Gln Leu Gly Phe Ser Val Glu Pro Lys Gly Glu Gly Ser Thr
   980 985 990
   Thr Val Thr Val Val Val Thr Asp Gly Lys Asp Asn Ile Asn Val Ser
   995 1000 1005
   Phe Lys Val Asn Val Ser Thr Ala Ser Tyr Thr Ser Val Val Asn
   1010 1015 1020
   Ser Gly Asn Pro Val Lys Asp Phe Ala Pro Ala Val Pro Thr Ala
   1025 1030 1035
   Ala Thr Ala Lys Leu Pro Glu Phe Thr Ser Val Ala Lys Ala Gly
   1040 1045 1050
   Thr Ile Lys Val Gln Gly Phe Asp Ile Pro Leu Thr Leu Gly Ser
   1055 1060 1065
   Thr Gln Ala Gln Val Leu Glu Thr Leu Asn Asn Phe Ile Thr Asp
   1070 1075 1080
   Tyr Lys Ile Asn Ala Ser Ala Lys Leu Val Asp Lys Ala Ile Val
   1085 1090 1095
   Ile Ser Ser Thr Glu Thr Gly Asn Ala Ala Thr Leu Thr Val Glu
   1100 1105 1110
   Gly Asp Ser Thr Leu Glu Leu Val Ala Gly Thr Val Leu Gly Thr
   1115 1120 1125
   Val Ser Thr Thr Pro Gly Ser Val Gly Thr Ala Thr Pro Ala Lys
   1130 1135 1140
   Tyr Ala Val Lys Val Leu Asn Gly Val Ser Asn Gly Thr Lys Val
   1145 1150 1155
   Asp Phe Lys Phe Thr Ile Gly Ala Lys Thr Val Thr Val Thr Val
   1160 1165 1170
   Asp Ala Thr Ala Gly Ala Lys Ala Val Ser Asp Val Val Asp Thr
   1175 1180 1185
   Leu Ala Ala Ala Thr Leu Asp Gly Tyr Ser Ile Thr Lys Asn Gly
   1190 1195 1200
   Asp Val Ile Glu Leu Thr Gln Thr Thr Pro Ala Thr Thr Thr Asp
   1205 1210 1215
   Lys Leu Val Val Glu Thr Lys Lys Ala Asn
   1220 1225
<210> 3
   <211> 3300
   <212> DNA
   <213> Lysinibacillus sphaericus
<220>
   <221> CDS
   <222> (1)..(3300)
<400> 3
   atg gct aac caa cca aag aaa tac aaa aaa ttc gta gcg acg gca gca        48
   Met Ala Asn Gln Pro Lys Lys Tyr Lys Lys Phe Val Ala Thr Ala Ala
   1               5                   10                  15
   acg gca aca tta gta gca tct gct atc gta cca gtg gtt tct gcc gca        96
   Thr Ala Thr Leu Val Ala Ser Ala Ile Val Pro Val Val Ser Ala Ala
               20                    25                    30
   gga ttt tca gat gta gca ggt aac gac cac gaa gta gca atc aac gca      144
   Gly Phe Ser Asp Val Ala Gly Asn Asp His Glu Val Ala Ile Asn Ala
           35                  40                  45
   ctt gct gat gca ggt atc atc aat gga tac gct gac ggc aca ttc aaa      192
   Leu Ala Asp Ala Gly Ile Ile Asn Gly Tyr Ala Asp Gly Thr Phe Lys
       50                  55                  60
   cca aac caa aca att aac cgc ggt caa gtg gtt aaa tta tta ggt cgt      240
   Pro Asn Gln Thr Ile Asn Arg Gly Gln Val Val Lys Leu Leu Gly Arg
   65                  70                  75                  80
   tat tta gaa gca caa ggc caa gaa att cca gct gac tgg aat tca aaa      288
   Tyr Leu Glu Ala Gln Gly Gln Glu Ile Pro Ala Asp Trp Asn Ser Lys
                   85                  90                  95
   caa cgc ttc aac gat cta cca gta aca gct gaa gca gaa tta gta aaa      336
   Gln Arg Phe Asn Asp Leu Pro Val Thr Ala Glu Ala Glu Leu Val Lys
               100                 105                 110
   tat gct gca cta gca aaa gat gca ggc gta ttc aac ggt tca aac ggc      384
   Tyr Ala Ala Leu Ala Lys Asp Ala Gly Val Phe Asn Gly Ser Asn Gly
           115                 120                 125
   aac tta aac gct tca caa aca atg caa cgt caa caa atg gca gta gtt      432
   Asn Leu Asn Ala Ser Gln Thr Met Gln Arg Gln Gln Met Ala Val Val
       130                 135                 140
   tta gta cgt gct att aaa gaa atc gct ggc gta gac tta gta gct gag      480
   Leu Val Arg Ala Ile Lys Glu Ile Ala Gly Val Asp Leu Val Ala Glu
   145                 150                 155                 160
   tac aaa aaa gcg aat ttc gta aca gaa att ggt gac tta gac aaa gct      528
   Tyr Lys Lys Ala Asn Phe Val Thr Glu Ile Gly Asp Leu Asp Lys Ala
                   165                 170                 175
   tac tct gca gaa caa cgc aca gcg atc gtt gct tta gaa tat gcg ggt      576
   Tyr Ser Ala Glu Gln Arg Thr Ala Ile Val Ala Leu Glu Tyr Ala Gly
               180                 185                 190
   att aca aat gtt gct cac ttc aac cca ggt aac agc gta aca cgc gga      624
   Ile Thr Asn Val Ala His Phe Asn Pro Gly Asn Ser Val Thr Arg Gly
           195                 200                 205
   caa ttc gct tca ttc tta tac cgc aca atc gaa aac gtg gta aac gct      672
   Gln Phe Ala Ser Phe Leu Tyr Arg Thr Ile Glu Asn Val Val Asn Ala
       210                 215                 220
   cct gaa gct ggc gct gcg gca gtg aaa gct gta aac aac aca act gtt      720
   Pro Glu Ala Gly Ala Ala Ala Val Lys Ala Val Asn Asn Thr Thr Val
   225                 230                 235                 240
   gaa gta aca ttc gac gaa gaa gta gac aac gta caa gct ctt aac ttc      768
   Glu Val Thr Phe Asp Glu Glu Val Asp Asn Val Gln Ala Leu Asn Phe
                   245                 250                 255
   tta atc tct gat tta gaa gtg aaa aac gca gct gta aaa caa act aac      816
   Leu Ile Ser Asp Leu Glu Val Lys Asn Ala Ala Val Lys Gln Thr Asn
               260                 265                 270
   aaa aaa gtt gtt gtt tta act act gca cct caa act gct gac aaa gag      864
   Lys Lys Val Val Val Leu Thr Thr Ala Pro Gln Thr Ala Asp Lys Glu
           275                 280                 285
   tac act gta tct ctt ggc gaa gaa aaa att ggt act ttc aaa ggt atc      912
   Tyr Thr Val Ser Leu Gly Glu Glu Lys Ile Gly Thr Phe Lys Gly Ile
       290                 295                 300
   gca gct gta gtt cct act aaa gta gat tta gta gaa aaa tca gtt caa      960
   Ala Ala Val Val Pro Thr Lys Val Asp Leu Val Glu Lys Ser Val Gln
   305                 310                 315                 320
   ggt aaa ctt ggc caa caa gta aca ctg aaa gca caa gta aca gtt gct     1008
   Gly Lys Leu Gly Gln Gln Val Thr Leu Lys Ala Gln Val Thr Val Ala
                   325                 330                 335
   gaa ggt caa aca aaa gct ggt att cca gta act ttc ttc atc cca ggt     1056
   Glu Gly Gln Thr Lys Ala Gly Ile Pro Val Thr Phe Phe Ile Pro Gly
               340                 345                 350
   tct gct aat ggt gtg aaa tct cca gta aca gtt gaa gct gta aca gat     1104
   Ser Ala Asn Gly Val Lys Ser Pro Val Thr Val Glu Ala Val Thr Asp
           355                 360                 365
   gaa aac ggt gtt gct tct tat act tac act cgt tac gct gca aca aac     1152
   Glu Asn Gly Val Ala Ser Tyr Thr Tyr Thr Arg Tyr Ala Ala Thr Asn
       370                 375                 380
   gac aca gta act gta tac gcg aac ggt gac cgt tct aaa ttc tca act     1200
   Asp Thr Val Thr Val Tyr Ala Asn Gly Asp Arg Ser Lys Phe Ser Thr
   385                 390                 395                 400
   ggt tat gtc ttc tgg gct gtt gat caa cat cta acg att act gaa gta     1248
   Gly Tyr Val Phe Trp Ala Val Asp Gln His Leu Thr Ile Thr Glu Val
                   405                 410                 415
   aca act ggt aat aca atc aac aac gga gct aac aaa acg tat aaa gtc     1296
   Thr Thr Gly Asn Thr Ile Asn Asn Gly Ala Asn Lys Thr Tyr Lys Val
               420                 425                 430
   act tac aaa cac ccg gaa act ggt aga ccg gta tca ggt aaa gta cta     1344
   Thr Tyr Lys His Pro Glu Thr Gly Arg Pro Val Ser Gly Lys Val Leu
           435                 440                 445
   aac gtt tct gta ctt gaa aac att gat gta acc gtt gat aaa tta caa     1392
   Asn Val Ser Val Leu Glu Asn Ile Asp Val Thr Val Asp Lys Leu Gln
       450                 455                 460
   aac gtt acg gtg aat ggt gtt gcg gta gtt caa acg agc gaa aat aac     1440
   Asn Val Thr Val Asn Gly Val Ala Val Val Gln Thr Ser Glu Asn Asn
   465                 470                 475                 480
   atg cga gct gca caa atc aca act gat tca aaa ggt gaa gca aca ttc     1488
   Met Arg Ala Ala Gln Ile Thr Thr Asp Ser Lys Gly Glu Ala Thr Phe
                   485                 490                 495
   aca att tca ggt tct aac gct aca gtt act cca gta gta ttt gaa gct     1536
   Thr Ile Ser Gly Ser Asn Ala Thr Val Thr Pro Val Val Phe Glu Ala
               500                 505                 510
   gct tca act aat gtt gca aac act tac tct cca aaa tat aca gca gat     1584
   Ala Ser Thr Asn Val Ala Asn Thr Tyr Ser Pro Lys Tyr Thr Ala Asp
           515                 520                 525
   aca cta caa gct gtt gcg tct aaa ata aca ttt ggt gct att caa gct     1632
   Thr Leu Gln Ala Val Ala Ser Lys Ile Thr Phe Gly Ala Ile Gln Ala
       530                 535                 540
   gct tat aca att gaa gta act cgt gat ggt ggt gaa act gct gct act     1680
   Ala Tyr Thr Ile Glu Val Thr Arg Asp Gly Gly Glu Thr Ala Ala Thr
   545                 550                 555                 560
   gga gtt tta aac gga cgt aaa tac aat gta gtg gtt aaa gat aaa gat     1728
   Gly Val Leu Asn Gly Arg Lys Tyr Asn Val Val Val Lys Asp Lys Asp
                   565                 570                 575
   ggt aaa gta gct gct aac gaa att gtt aac gtg gca ttt aac gaa gat      1776
   Gly Lys Val Ala Ala Asn Glu Ile Val Asn Val Ala Phe Asn Glu Asp
               580                 585                 590
   att gat ggc gta att tct aca gtt act tct gct caa ttc gta aaa atc      1824
   Ile Asp Gly Val Ile Ser Thr Val Thr Ser Ala Gln Phe Val Lys Ile
           595                 600                 605
   gaa aac ggt aaa caa gtt ggc tac aat ggt aag aaa att acg gtt aca      1872
   Glu Asn Gly Lys Gln Val Gly Tyr Asn Gly Lys Lys Ile Thr Val Thr
       610                 615                 620
   aca aac tct aaa ggt gaa gca agc ttc gtc atc gga agt gac gct gta      1920
   Thr Asn Ser Lys Gly Glu Ala Ser Phe Val Ile Gly Ser Asp Ala Val
   625                 630                 635                 640
   aat tct tac gct aca cca atc gtt tgg att gac atc aat aac caa agt      1968
   Asn Ser Tyr Ala Thr Pro Ile Val Trp Ile Asp Ile Asn Asn Gln Ser
                   645                 650                 655
   ggt aaa gat gca aac tta gac aaa ggt gag cct tca gca att gca cca      2016
   Gly Lys Asp Ala Asn Leu Asp Lys Gly Glu Pro Ser Ala Ile Ala Pro
               660                 665                 670
   att tca tac ttc caa gca cca tat tta gat ggt tct aaa tta gta tct      2064
   Ile Ser Tyr Phe Gln Ala Pro Tyr Leu Asp Gly Ser Lys Leu Val Ser
           675                 680                 685
   tac aaa ggt aca act gaa act gac aaa ttt gtt agt gga gaa act gca      2112
   Tyr Lys Gly Thr Thr Glu Thr Asp Lys Phe Val Ser Gly Glu Thr Ala
       690                 695                 700
   aca ttt aaa gta caa ctt acg aac caa agt ggt aaa gtc gta cag aat      2160
   Thr Phe Lys Val Gln Leu Thr Asn Gln Ser Gly Lys Val Val Gln Asn
   705                 710                 715                 720
   tct agt tac aca aca aag gat gtt acg tac act gtt tac aac act ggt      2208
   Ser Ser Tyr Thr Thr Lys Asp Val Thr Tyr Thr Val Tyr Asn Thr Gly
                   725                 730                 735
   gct aat aat gta aaa gtt ggc gat gtt gaa atc gct cca aac cgc gtg      2256
   Ala Asn Asn Val Lys Val Gly Asp Val Glu Ile Ala Pro Asn Arg Val
               740                 745                 750
   cat aca tta gtt gcc gca aac ggt gaa atc gac gtt act act gta gat      2304
   His Thr Leu Val Ala Ala Asn Gly Glu Ile Asp Val Thr Thr Val Asp
           755                 760                 765
   ggt aaa tct tct tct gtg aaa gtt ctt gct act ggt gta gcg aaa gac      2352
   Gly Lys Ser Ser Ser Val Lys Val Leu Ala Thr Gly Val Ala Lys Asp
       770                 775                 780
   gga aac aaa gaa ttc gca ttt act tca aaa gaa gca act gca aca ttc      2400
   Gly Asn Lys Glu Phe Ala Phe Thr Ser Lys Glu Ala Thr Ala Thr Phe
   785                 790                 795                 800
   act tct tcg aaa gaa gta acg aac cta ttt act ggt gat att cat tca      2448
   Thr Ser Ser Lys Glu Val Thr Asn Leu Phe Thr Gly Asp Ile His Ser
                   805                 810                 815
   ttc gat aca gaa aaa gaa act atc gta ttt act gat ggt tcc gta cta      2496
   Phe Asp Thr Glu Lys Glu Thr Ile Val Phe Thr Asp Gly Ser Val Leu
               820                 825                 830
   aaa gat gcg gtg aaa tac gct ggt gaa aca gat aaa aaa tat gaa tat      2544
   Lys Asp Ala Val Lys Tyr Ala Gly Glu Thr Asp Lys Lys Tyr Glu Tyr
           835                 840                 845
   aga aat ata aat cgt gca cca att tct aaa gca gag ttt gta aat att      2592
   Arg Asn Ile Asn Arg Ala Pro Ile Ser Lys Ala Glu Phe Val Asn Ile
       850                 855                 860
   ctt aaa ggg gca att aca ggg gta aaa gta act tat gaa gtg aaa gat      2640
   Leu Lys Gly Ala Ile Thr Gly Val Lys Val Thr Tyr Glu Val Lys Asp
   865                 870                 875                 880
   aat acg gta ata ttc cat gtt ctt tct gta aat gct ggt ggt act aaa      2688
   Asn Thr Val Ile Phe His Val Leu Ser Val Asn Ala Gly Gly Thr Lys
                   885                 890                 895
   ccg gtt gat att att gga gat aaa ata tct gat gct act gct gtt aca      2736
   Pro Val Asp Ile Ile Gly Asp Lys Ile Ser Asp Ala Thr Ala Val Thr
               900                 905                 910
   acg gca aaa ggt aaa gtt aca gta aca cct aat att gct aca ctt tca      2784
   Thr Ala Lys Gly Lys Val Thr Val Thr Pro Asn Ile Ala Thr Leu Ser
           915                 920                 925
   act gat acg gcg gaa aat aaa cgt tta gct gta caa tca aat gtt gta      2832
   Thr Asp Thr Ala Glu Asn Lys Arg Leu Ala Val Gln Ser Asn Val Val
       930                 935                 940
   gct gaa att aac gac cca tca gta act gta agt gta gta gaa act tcg      2880
   Ala Glu Ile Asn Asp Pro Ser Val Thr Val Ser Val Val Glu Thr Ser
   945                 950                 955                 960
   aca gta gga act tat act gtg acg tta act aaa ggc gca agc act gct      2928
   Thr Val Gly Thr Tyr Thr Val Thr Leu Thr Lys Gly Ala Ser Thr Ala
                   965                 970                 975
   aca gta atg gtt aca cca aaa ttt ggt caa aaa tta act gct gaa tcg      2976
   Thr Val Met Val Thr Pro Lys Phe Gly Gln Lys Leu Thr Ala Glu Ser
               980                 985                 990
   att act tct tca ctt gca gtg aat  aca aat gga aaa gat  tta aat ctt    3024
   Ile Thr Ser Ser Leu Ala Val Asn  Thr Asn Gly Lys Asp  Leu Asn Leu
           995                 1000                 1005
   aat ctt  aca tct act ttt gct  ggt ttc aca atc gct  aat gta aat      3069
   Asn Leu  Thr Ser Thr Phe Ala  Gly Phe Thr Ile Ala  Asn Val Asn
       1010                 1015                 1020
   cca gat  aga gat aat tca tta  gac att act act tct  gtt aat aat      3114
   Pro Asp  Arg Asp Asn Ser Leu  Asp Ile Thr Thr Ser  Val Asn Asn
       1025                 1030                 1035
   aat gtg  att act ata tct aaa  tca cct tct gat tct  act gaa gct      3159
   Asn Val  Ile Thr Ile Ser Lys  Ser Pro Ser Asp Ser  Thr Glu Ala
       1040                 1045                 1050
   gca caa  tta atc gag aag act  tta aaa att tat ttc  gac gct caa      3204
   Ala Gln  Leu Ile Glu Lys Thr  Leu Lys Ile Tyr Phe  Asp Ala Gln
       1055                 1060                 1065
   gct act  ggt tat gcc aaa gaa  agc ttt gta gca act  att aca att      3249
   Ala Thr  Gly Tyr Ala Lys Glu  Ser Phe Val Ala Thr  Ile Thr Ile
       1070                 1075                 1080
   gat gat  gta acg aac cgt act  tta gct att aca gta  aag cca act      3294
   Asp Asp  Val Thr Asn Arg Thr  Leu Ala Ile Thr Val  Lys Pro Thr
       1085                 1090                 1095
   cca taa                                                              3300
   Pro
<210> 4
   <211> 1099
   <212> PRT
   <213> Lysinibacillus sphaericus
<400> 4
   Met Ala Asn Gln Pro Lys Lys Tyr Lys Lys Phe Val Ala Thr Ala Ala
   1               5                   10                  15
   Thr Ala Thr Leu Val Ala Ser Ala Ile Val Pro Val Val Ser Ala Ala
               20                  25                  30
   Gly Phe Ser Asp Val Ala Gly Asn Asp His Glu Val Ala Ile Asn Ala
           35                  40                  45
   Leu Ala Asp Ala Gly Ile Ile Asn Gly Tyr Ala Asp Gly Thr Phe Lys
       50                  55                  60
   Pro Asn Gln Thr Ile Asn Arg Gly Gln Val Val Lys Leu Leu Gly Arg
   65                  70                  75                  80
   Tyr Leu Glu Ala Gln Gly Gln Glu Ile Pro Ala Asp Trp Asn Ser Lys
                   85                  90                  95
   Gln Arg Phe Asn Asp Leu Pro Val Thr Ala Glu Ala Glu Leu Val Lys
               100                 105                 110
   Tyr Ala Ala Leu Ala Lys Asp Ala Gly Val Phe Asn Gly Ser Asn Gly
           115                 120                 125
   Asn Leu Asn Ala Ser Gln Thr Met Gln Arg Gln Gln Met Ala Val Val
       130                 135                 140
   Leu Val Arg Ala Ile Lys Glu Ile Ala Gly Val Asp Leu Val Ala Glu
   145                 150                 155                 160
   Tyr Lys Lys Ala Asn Phe Val Thr Glu Ile Gly Asp Leu Asp Lys Ala
                   165                 170                 175
   Tyr Ser Ala Glu Gln Arg Thr Ala Ile Val Ala Leu Glu Tyr Ala Gly
               180                 185                 190
   Ile Thr Asn Val Ala His Phe Asn Pro Gly Asn Ser Val Thr Arg Gly
           195                 200                 205
   Gln Phe Ala Ser Phe Leu Tyr Arg Thr Ile Glu Asn Val Val Asn Ala
       210                 215                 220
   Pro Glu Ala Gly Ala Ala Ala Val Lys Ala Val Asn Asn Thr Thr Val
   225                 230                 235                 240
   Glu Val Thr Phe Asp Glu Glu Val Asp Asn Val Gln Ala Leu Asn Phe
                   245                 250                 255
   Leu Ile Ser Asp Leu Glu Val Lys Asn Ala Ala Val Lys Gln Thr Asn
               260                 265                 270
   Lys Lys Val Val Val Leu Thr Thr Ala Pro Gln Thr Ala Asp Lys Glu
           275                 280                 285
   Tyr Thr Val Ser Leu Gly Glu Glu Lys Ile Gly Thr Phe Lys Gly Ile
       290                 295                 300
   Ala Ala Val Val Pro Thr Lys Val Asp Leu Val Glu Lys Ser Val Gln
   305                 310                 315                 320
   Gly Lys Leu Gly Gln Gln Val Thr Leu Lys Ala Gln Val Thr Val Ala
                   325                 330                 335
   Glu Gly Gln Thr Lys Ala Gly Ile Pro Val Thr Phe Phe Ile Pro Gly
               340                 345                 350
   Ser Ala Asn Gly Val Lys Ser Pro Val Thr Val Glu Ala Val Thr Asp
           355                 360                 365
   Glu Asn Gly Val Ala Ser Tyr Thr Tyr Thr Arg Tyr Ala Ala Thr Asn
       370                 375                 380
   Asp Thr Val Thr Val Tyr Ala Asn Gly Asp Arg Ser Lys Phe Ser Thr
   385                 390                 395                 400
   Gly Tyr Val Phe Trp Ala Val Asp Gln His Leu Thr Ile Thr Glu Val
                   405                 410                 415
   Thr Thr Gly Asn Thr Ile Asn Asn Gly Ala Asn Lys Thr Tyr Lys Val
               420                 425                 430
   Thr Tyr Lys His Pro Glu Thr Gly Arg Pro Val Ser Gly Lys Val Leu
           435                 440                 445
   Asn Val Ser Val Leu Glu Asn Ile Asp Val Thr Val Asp Lys Leu Gln
       450                 455                 460
   Asn Val Thr Val Asn Gly Val Ala Val Val Gln Thr Ser Glu Asn Asn
   465                 470                 475                 480
   Met Arg Ala Ala Gln Ile Thr Thr Asp Ser Lys Gly Glu Ala Thr Phe
                   485                 490                 495
   Thr Ile Ser Gly Ser Asn Ala Thr Val Thr Pro Val Val Phe Glu Ala
               500                 505                 510
   Ala Ser Thr Asn Val Ala Asn Thr Tyr Ser Pro Lys Tyr Thr Ala Asp
           515                 520                 525
   Thr Leu Gln Ala Val Ala Ser Lys Ile Thr Phe Gly Ala Ile Gln Ala
   530 535 540
   Ala Tyr Thr Ile Glu Val Thr Arg Asp Gly Gly Glu Thr Ala Ala Thr
   545 550 555 560
   Gly Val Leu Asn Gly Arg Lys Tyr Asn Val Val Val Lys Asp Lys Asp
   565 570 575
   Gly Lys Val Ala Ala Asn Glu Ile Val Asn Val Ala Phe Asn Glu Asp
   580 585 590
   Ile Asp Gly Val Ile Ser Thr Val Thr Ser Ala Gln Phe Val Lys Ile
   595 600 605
   Glu Asn Gly Lys Gln Val Gly Tyr Asn Gly Lys Lys Ile Thr Val Thr
   610 615 620
   Thr Asn Ser Lys Gly Glu Ala Ser Phe Val Ile Gly Ser Asp Ala Val
   625 630 635 640
   Asn Ser Tyr Ala Thr Pro Ile Val Trp Ile Asp Ile Asn Asn Gln Ser
   645 650 655
   Gly Lys Asp Ala Asn Leu Asp Lys Gly Glu Pro Ser Ala Ile Ala Pro
   660 665 670
   Ile Ser Tyr Phe Gln Ala Pro Tyr Leu Asp Gly Ser Lys Leu Val Ser
   675 680 685
   Tyr Lys Gly Thr Thr Glu Thr Asp Lys Phe Val Ser Gly Glu Thr Ala
   690 695 700
   Thr Phe Lys Val Gln Leu Thr Asn Gln Ser Gly Lys Val Val Gln Asn
   705 710 715 720
   Ser Ser Tyr Thr Thr Lys Asp Val Thr Tyr Thr Val Tyr Asn Thr Gly
   725 730 735
   Ala Asn Asn Val Lys Val Gly Asp Val Glu Ile Ala Pro Asn Arg Val
   740 745 750
   His Thr Leu Val Ala Ala Asn Gly Glu Ile Asp Val Thr Thr Val Asp
   755 760 765
   Gly Lys Ser Ser Ser Val Lys Val Leu Ala Thr Gly Val Ala Lys Asp
   770 775 780
   Gly Asn Lys Glu Phe Ala Phe Thr Ser Lys Glu Ala Thr Ala Thr Phe
   785 790 795 800
   Thr Ser Ser Lys Glu Val Thr Asn Leu Phe Thr Gly Asp Ile His Ser
                   805                 810                 815
   Phe Asp Thr Glu Lys Glu Thr Ile Val Phe Thr Asp Gly Ser Val Leu
               820                 825                 830
   Lys Asp Ala Val Lys Tyr Ala Gly Glu Thr Asp Lys Lys Tyr Glu Tyr
           835                 840                 845
   Arg Asn Ile Asn Arg Ala Pro Ile Ser Lys Ala Glu Phe Val Asn Ile
       850                 855                 860
   Leu Lys Gly Ala Ile Thr Gly Val Lys Val Thr Tyr Glu Val Lys Asp
   865                 870                 875                 880
   Asn Thr Val Ile Phe His Val Leu Ser Val Asn Ala Gly Gly Thr Lys
                   885                 890                 895
   Pro Val Asp Ile Ile Gly Asp Lys Ile Ser Asp Ala Thr Ala Val Thr
               900                 905                 910
   Thr Ala Lys Gly Lys Val Thr Val Thr Pro Asn Ile Ala Thr Leu Ser
           915                 920                 925
   Thr Asp Thr Ala Glu Asn Lys Arg Leu Ala Val Gln Ser Asn Val Val
       930                 935                 940
   Ala Glu Ile Asn Asp Pro Ser Val Thr Val Ser Val Val Glu Thr Ser
   945                 950                 955                 960
   Thr Val Gly Thr Tyr Thr Val Thr Leu Thr Lys Gly Ala Ser Thr Ala
                   965                 970                 975
   Thr Val Met Val Thr Pro Lys Phe Gly Gln Lys Leu Thr Ala Glu Ser
               980                 985                 990
   Ile Thr Ser Ser Leu Ala Val Asn  Thr Asn Gly Lys Asp  Leu Asn Leu
           995                 1000                1005
   Asn Leu  Thr Ser Thr Phe Ala  Gly Phe Thr Ile Ala  Asn Val Asn
       1010                 1015                 1020
   Pro Asp  Arg Asp Asn Ser Leu  Asp Ile Thr Thr Ser  Val Asn Asn
       1025                 1030                 1035
   Asn Val  Ile Thr Ile Ser Lys  Ser Pro Ser Asp Ser  Thr Glu Ala
       1040                 1045                 1050
   Ala Gln  Leu Ile Glu Lys Thr  Leu Lys Ile Tyr Phe  Asp Ala Gln
       1055                 1060                 1065
   Ala Thr  Gly Tyr Ala Lys Glu  Ser Phe Val Ala Thr  Ile Thr Ile
       1070                 1075                 1080
   Asp Asp  Val Thr Asn Arg Thr  Leu Ala Ile Thr Val  Lys Pro Thr
       1085                 1090                 1095
   Pro
<210> 5
   <211> 3714
   <212> DNA
   <213> Lysinibacillus sphaericus
<220>
   <221> CDS
   <222> (1)..(3714)
<400> 5
   atg gct aac caa cca aag aaa tac aaa aaa ttc gta gct acg gct gca      48
   Met Ala Asn Gln Pro Lys Lys Tyr Lys Lys Phe Val Ala Thr Ala Ala
   1               5                   10                  15
   acg gct act tta gta gct tct gct atc gta cca gtg gct tct gca gca      96
   Thr Ala Thr Leu Val Ala Ser Ala Ile Val Pro Val Ala Ser Ala Ala
               20                  25                  30
   gga ttc tca gat gta gca ggt aac gac cac gaa gta gca atc aac gca     144
   Gly Phe Ser Asp Val Ala Gly Asn Asp His Glu Val Ala Ile Asn Ala
           35                  40                  45
   ctt gct gat gca ggt atc atc aat gga tac gct gac ggc tca ttc aaa     192
   Leu Ala Asp Ala Gly Ile Ile Asn Gly Tyr Ala Asp Gly Ser Phe Lys
       50                  55                  60
   cca aac caa aca att aac cgc ggt caa gtg gtt aaa tta tta ggt cgt     240
   Pro Asn Gln Thr Ile Asn Arg Gly Gln Val Val Lys Leu Leu Gly Arg
   65                  70                  75                  80
   tat tta gaa gca caa ggc caa gaa att cca gct gac tgg aat tca aaa     288
   Tyr Leu Glu Ala Gln Gly Gln Glu Ile Pro Ala Asp Trp Asn Ser Lys
                   85                  90                  95
   caa cgc ttc aac gat cta cca gta aca gct gaa gaa gaa tta gtt aaa     336
   Gln Arg Phe Asn Asp Leu Pro Val Thr Ala Glu Glu Glu Leu Val Lys
               100                 105                 110
   tat gct gca ctt gca aaa gat gca ggc gta ttc aac ggt tca aat ggc     384
   Tyr Ala Ala Leu Ala Lys Asp Ala Gly Val Phe Asn Gly Ser Asn Gly
           115                 120                 125
   aac cta aac gct tca caa aca atg caa cgt caa caa atg gca gta gtt     432
   Asn Leu Asn Ala Ser Gln Thr Met Gln Arg Gln Gln Met Ala Val Val
       130                 135                 140
   tta gta cgt gct att aaa gaa atc gca ggc gta gac tta gta gct gag     480
   Leu Val Arg Ala Ile Lys Glu Ile Ala Gly Val Asp Leu Val Ala Glu
   145                 150                 155                 160
   tac aaa aaa gcg aac ttc gta aca gaa att ggt gac tta gac aaa gct     528
   Tyr Lys Lys Ala Asn Phe Val Thr Glu Ile Gly Asp Leu Asp Lys Ala
                   165                 170                 175
   tac tct gca gaa caa cgc aca gcg atc gtt gct tta gaa tat gcg ggt     576
   Tyr Ser Ala Glu Gln Arg Thr Ala Ile Val Ala Leu Glu Tyr Ala Gly
               180                 185                 190
   att aca aat gtt gct cac ttc aac cca ggt aac agc gta aca cgc gga      624
   Ile Thr Asn Val Ala His Phe Asn Pro Gly Asn Ser Val Thr Arg Gly
           195                 200                 205
   caa ttc gct tca ttc tta tac cgc aca atc gaa aac gtt gta aac aac      672
   Gln Phe Ala Ser Phe Leu Tyr Arg Thr Ile Glu Asn Val Val Asn Asn
       210                 215                 220
   cca gaa gct ggt gtt gcg gca gta aaa gct atc aac aac aca act gtt      720
   Pro Glu Ala Gly Val Ala Ala Val Lys Ala Ile Asn Asn Thr Thr Val
   225                 230                 235                 240
   gaa gta aca ttc gac gaa gaa gta gac aac gta caa gct ctt aac ttc      768
   Glu Val Thr Phe Asp Glu Glu Val Asp Asn Val Gln Ala Leu Asn Phe
                   245                 250                 255
   tta atc tct gat ctt gaa gtg aaa aac gca gct gtt aaa tta aca aac      816
   Leu Ile Ser Asp Leu Glu Val Lys Asn Ala Ala Val Lys Leu Thr Asn
               260                 265                 270
   aaa aaa gtt gtt gta tta aca act gct gct caa aca gct gac aaa gag      864
   Lys Lys Val Val Val Leu Thr Thr Ala Ala Gln Thr Ala Asp Lys Glu
           275                 280                 285
   tac act gta tct ctt ggc gaa gaa aaa atc ggt aca ttc aaa ggt atc      912
   Tyr Thr Val Ser Leu Gly Glu Glu Lys Ile Gly Thr Phe Lys Gly Ile
       290                 295                 300
   gca gct gta gtt cct act aaa gta gat tta gta gaa aaa tca gtt caa      960
   Ala Ala Val Val Pro Thr Lys Val Asp Leu Val Glu Lys Ser Val Gln
   305                 310                 315                 320
   ggt aaa ctt ggc caa caa gta aca ctt aaa gca caa gta act gtt gct     1008
   Gly Lys Leu Gly Gln Gln Val Thr Leu Lys Ala Gln Val Thr Val Ala
                   325                 330                 335
   gaa ggt caa aca aaa gct ggt att cca gta act ttc ttc atc cca ggt     1056
   Glu Gly Gln Thr Lys Ala Gly Ile Pro Val Thr Phe Phe Ile Pro Gly
               340                 345                 350
   tct gct aat ggc gtg aaa act cca gta aca gtt gaa gct gta aca aac     1104
   Ser Ala Asn Gly Val Lys Thr Pro Val Thr Val Glu Ala Val Thr Asn
           355                 360                 365
   gaa gaa ggt atc gca act tac tct tac act cgt tat gct gca aca aac     1152
   Glu Glu Gly Ile Ala Thr Tyr Ser Tyr Thr Arg Tyr Ala Ala Thr Asn
       370                 375                 380
   gac act gta act gta tat gca aac ggt gac cgt tct aaa ttc tca act     1200
   Asp Thr Val Thr Val Tyr Ala Asn Gly Asp Arg Ser Lys Phe Ser Thr
   385                 390                 395                 400
   ggt tat gta ttc tgg gca gtt gac caa act tta gaa atc act gaa gta     1248
   Gly Tyr Val Phe Trp Ala Val Asp Gln Thr Leu Glu Ile Thr Glu Val
                   405                 410                 415
   aca act ggt gct act atc aac aac ggc gct aac aaa act tac aaa gtt     1296
   Thr Thr Gly Ala Thr Ile Asn Asn Gly Ala Asn Lys Thr Tyr Lys Val
               420                 425                 430
   act tac aaa cac cca gaa act ggt aaa cca gta tca ggt aaa gta ctt     1344
   Thr Tyr Lys His Pro Glu Thr Gly Lys Pro Val Ser Gly Lys Val Leu
           435                 440                 445
   aac gtt tca gta aaa gaa aac att gac gta act gtt gat aaa tta caa     1392
   Asn Val Ser Val Lys Glu Asn Ile Asp Val Thr Val Asp Lys Leu Gln
       450                 455                 460
   aac gtt acg gtg aat ggt att gct gta gtt caa aca agc gat aac aac     1440
   Asn Val Thr Val Asn Gly Ile Ala Val Val Gln Thr Ser Asp Asn Asn
   465                 470                 475                 480
   aca cgt gct gct caa atc aca act gac tct aaa ggt gaa gca aca ttc      1488
   Thr Arg Ala Ala Gln Ile Thr Thr Asp Ser Lys Gly Glu Ala Thr Phe
                   485                 490                 495
   aca gtt tca ggt tct aac gct gaa gta act cca gtg gta ttt gaa gca      1536
   Thr Val Ser Gly Ser Asn Ala Glu Val Thr Pro Val Val Phe Glu Ala
               500                 505                 510
   act cca gta caa gtt act aac aca aat ggt gca gta aca aca act agc      1584
   Thr Pro Val Gln Val Thr Asn Thr Asn Gly Ala Val Thr Thr Thr Ser
           515                 520                 525
   tac act caa aaa tac act gct gat att tta caa gct tct gct gcg aaa      1632
   Tyr Thr Gln Lys Tyr Thr Ala Asp Ile Leu Gln Ala Ser Ala Ala Lys
       530                 535                 540
   gta aca ttt ggt gct gtt caa gct gct tac act ctt gaa gta act cgc      1680
   Val Thr Phe Gly Ala Val Gln Ala Ala Tyr Thr Leu Glu Val Thr Arg
   545                 550                 555                 560
   gat ggt ggc gaa gtt gcg gca act ggt gtt gaa aac ggc cgt aaa tat      1728
   Asp Gly Gly Glu Val Ala Ala Thr Gly Val Glu Asn Gly Arg Lys Tyr
                   565                 570                 575
   aac tta gta gta aaa gat aaa gat ggt aaa tta gct gct aac gaa aca      1776
   Asn Leu Val Val Lys Asp Lys Asp Gly Lys Leu Ala Ala Asn Glu Thr
               580                 585                 590
   gta aac gtt gca ttt aac gaa gat att gac gga gta att tct aca gtt      1824
   Val Asn Val Ala Phe Asn Glu Asp Ile Asp Gly Val Ile Ser Thr Val
           595                 600                 605
   act tct gca gaa ttc gta aaa gta gaa aac gat aaa caa gtt cgc tac      1872
   Thr Ser Ala Glu Phe Val Lys Val Glu Asn Asp Lys Gln Val Arg Tyr
       610                 615                 620
   gaa ggc aaa aaa atc act gtt aaa aca aat tct aaa ggt gaa gca agc      1920
   Glu Gly Lys Lys Ile Thr Val Lys Thr Asn Ser Lys Gly Glu Ala Ser
   625                 630                 635                 640
   ttc gta att tca agt gaa gct gta aac act tat gct aca cca atc gct      1968
   Phe Val Ile Ser Ser Glu Ala Val Asn Thr Tyr Ala Thr Pro Ile Ala
                   645                 650                 655
   tgg atc gat atc aac aac caa agc gct aaa gat gca aac tta gac aaa      2016
   Trp Ile Asp Ile Asn Asn Gln Ser Ala Lys Asp Ala Asn Leu Asp Lys
               660                 665                 670
   ggt gaa cca tct gca gtt gca cca att tca tac ttc caa gct gaa tat      2064
   Gly Glu Pro Ser Ala Val Ala Pro Ile Ser Tyr Phe Gln Ala Glu Tyr
           675                 680                 685
   tta gat ggt tct aaa tta gta tct tac aaa gat aca act gaa act gat      2112
   Leu Asp Gly Ser Lys Leu Val Ser Tyr Lys Asp Thr Thr Glu Thr Asp
       690                 695                 700
   aaa ttc act ggt agc gaa act gca aca ttc aaa gta caa ctt aca aac      2160
   Lys Phe Thr Gly Ser Glu Thr Ala Thr Phe Lys Val Gln Leu Thr Asn
   705                 710                 715                 720
   caa agt ggt aaa gta gtt aaa aac tct ggt tat aca aca cca aat gtt      2208
   Gln Ser Gly Lys Val Val Lys Asn Ser Gly Tyr Thr Thr Pro Asn Val
                   725                 730                 735
   tct tac act gta tac aac act ggt gca aac aat gta aaa gtt gac ggt      2256
   Ser Tyr Thr Val Tyr Asn Thr Gly Ala Asn Asn Val Lys Val Asp Gly
               740                 745                 750
   gta gaa atc gca cca aac cgc gtg cat aca gta gtt gca aaa gac ggt      2304
   Val Glu Ile Ala Pro Asn Arg Val His Thr Val Val Ala Lys Asp Gly
           755                 760                 765
   gta atc aat gtt act aca gta gat aat aag tct tct tct gtt aaa gtt      2352
   Val Ile Asn Val Thr Thr Val Asp Asn Lys Ser Ser Ser Val Lys Val
       770                 775                 780
   ctt gct act ggt gta gct aaa caa aca aac ggt aac aaa gaa ttt gca      2400
   Leu Ala Thr Gly Val Ala Lys Gln Thr Asn Gly Asn Lys Glu Phe Ala
   785                 790                 795                 800
   ttc act tct aaa gaa gca act gca aca ttc aca gct act aat gaa gta      2448
   Phe Thr Ser Lys Glu Ala Thr Ala Thr Phe Thr Ala Thr Asn Glu Val
                   805                 810                 815
   tct aat cca tat act ggt gct gtt aaa cat tac aat aca gac aag aaa      2496
   Ser Asn Pro Tyr Thr Gly Ala Val Lys His Tyr Asn Thr Asp Lys Lys
               820                 825                 830
   act att aca ttc gat aat aaa gat gca att aaa tat gcg ggt gta aaa      2544
   Thr Ile Thr Phe Asp Asn Lys Asp Ala Ile Lys Tyr Ala Gly Val Lys
           835                 840                 845
   ggt aaa act tac aaa tac ttt ggt ctt ggt agc act cca att gca aat      2592
   Gly Lys Thr Tyr Lys Tyr Phe Gly Leu Gly Ser Thr Pro Ile Ala Asn
       850                 855                 860
   gca gat gca ttt atc gca aca tta agt ggt caa ggt gct ggt aac caa      2640
   Ala Asp Ala Phe Ile Ala Thr Leu Ser Gly Gln Gly Ala Gly Asn Gln
   865                 870                 875                 880
   gtt act gta act tat aaa gaa gaa gat gat gtt gta tca ttc tac att      2688
   Val Thr Val Thr Tyr Lys Glu Glu Asp Asp Val Val Ser Phe Tyr Ile
                   885                 890                 895
   att agt gtt gta aat ggt ggt att gga aat cct aca act gac cct gct      2736
   Ile Ser Val Val Asn Gly Gly Ile Gly Asn Pro Thr Thr Asp Pro Ala
               900                 905                 910
   cta gct aac ggt gta aca gga aca att gcg ttc acg aat aca tca ttc      2784
   Leu Ala Asn Gly Val Thr Gly Thr Ile Ala Phe Thr Asn Thr Ser Phe
           915                 920                 925
   aat gca agt gca aat caa aca att aca gtt aaa gat gct gat tta aat      2832
   Asn Ala Ser Ala Asn Gln Thr Ile Thr Val Lys Asp Ala Asp Leu Asn
       930                 935                 940
   gta aat gct act gta gct gat act aca act gtt aca att aca gat tct      2880
   Val Asn Ala Thr Val Ala Asp Thr Thr Thr Val Thr Ile Thr Asp Ser
   945                 950                 955                 960
   gct aaa act aca ttc aac atc act tta act gaa act ggg gtt aat act      2928
   Ala Lys Thr Thr Phe Asn Ile Thr Leu Thr Glu Thr Gly Val Asn Thr
                   965                 970                 975
   gga gaa ttc aca gga act ctt act tca gct caa cta gct tta tta aaa      2976
   Gly Glu Phe Thr Gly Thr Leu Thr Ser Ala Gln Leu Ala Leu Leu Lys
               980                 985                 990
   gat ggt gtg att aca gca act tat  aat gat gct aaa aat  gct aac aat      3024
   Asp Gly Val Ile Thr Ala Thr Tyr  Asn Asp Ala Lys Asn  Ala Asn Asn
           995                 1000                1005
   gtt gca  gct act gct act gct  act gct aca tta aat  aca aca gtt      3069
   Val Ala  Ala Thr Ala Thr Ala  Thr Ala Thr Leu Asn  Thr Thr Val
       1010                1015                1020
   ggc gct gta aac ttt gct gct aaa gca act act gaa tat tca gaa     3114
   Gly Ala Val Asn Phe Ala Ala Lys Ala Thr Thr Glu Tyr Ser Glu
       1025                1030                1035
   ggt gtt ggt aca gct gct aaa gtt gaa ggt agt act gta tta act     3159
   Gly Val Gly Thr Ala Ala Lys Val Glu Gly Ser Thr Val Leu Thr
       1040                1045                1050
   tca aaa gtt gat act ggt gat ctt gta tta gta gta gat ggt gtt     3204
   Ser Lys Val Asp Thr Gly Asp Leu Val Leu Val Val Asp Gly Val
       1055                1060                1065
   caa ttc aca act gtt gtt act gca att gat cct ggt act tca gct     3249
   Gln Phe Thr Thr Val Val Thr Ala Ile Asp Pro Gly Thr Ser Ala
       1070                1075                1080
   aca gct agt tta act gca gta gtt gct gaa atc aat gca gca gct     3294
   Thr Ala Ser Leu Thr Ala Val Val Ala Glu Ile Asn Ala Ala Ala
       1085                1090                1095
   aaa aaa gtt gga ttc aca gct gat gtt gca act gtt gat gct gat     3339
   Lys Lys Val Gly Phe Thr Ala Asp Val Ala Thr Val Asp Ala Asp
       1100                1105                1110
   aaa atc gtt ctt aca agc cca aca aaa ggt aca tct tca tct gta     3384
   Lys Ile Val Leu Thr Ser Pro Thr Lys Gly Thr Ser Ser Ser Val
       1115                1120                1125
   gaa gtt aaa gat tta gga aca act act gga tta ggt tta act aaa     3429
   Glu Val Lys Asp Leu Gly Thr Thr Thr Gly Leu Gly Leu Thr Lys
       1130                1135                1140
   gca cct gaa gta aaa ggt act gct ggt gct gct gta gca act caa     3474
   Ala Pro Glu Val Lys Gly Thr Ala Gly Ala Ala Val Ala Thr Gln
       1145                1150                1155
   tgg aag ttc aca gta act act act cca gct gtt ggt gaa act att     3519
   Trp Lys Phe Thr Val Thr Thr Thr Pro Ala Val Gly Glu Thr Ile
       1160                1165                1170
   aca gtt aaa gtt gga act aaa gaa gct tct cac aaa gtt gtt gca     3564
   Thr Val Lys Val Gly Thr Lys Glu Ala Ser His Lys Val Val Ala
       1175                1180                1185
   ggt aac gat tta gct gct att gca act gct tta aat act gct atc     3609
   Gly Asn Asp Leu Ala Ala Ile Ala Thr Ala Leu Asn Thr Ala Ile
       1190                1195                1200
   ggt gct gac tat aat atc gca ctt gtt act ggt tct aat gta att     3654
   Gly Ala Asp Tyr Asn Ile Ala Leu Val Thr Gly Ser Asn Val Ile
       1205                1210                1215
   aca gtt aca caa gct act cca gct aaa aca act gat gaa tta tca     3699
   Thr Val Thr Gln Ala Thr Pro Ala Lys Thr Thr Asp Glu Leu Ser
       1220                1225                1230
   gtt act gtt aca aaa                                             3714
   Val Thr Val Thr Lys
       1235
<210> 6
   <211> 1238
   <212> PRT
   <213> Lysinibacillus sphaericus
<400> 6
   Met Ala Asn Gln Pro Lys Lys Tyr Lys Lys Phe Val Ala Thr Ala Ala
   1 5 10 15
   Thr Ala Thr Leu Val Ala Ser Ala Ile Val Pro Val Ala Ser Ala Ala
   20 25 30
   Gly Phe Ser Asp Val Ala Gly Asn Asp His Glu Val Ala Ile Asn Ala
   35 40 45
   Leu Ala Asp Ala Gly Ile Ile Asn Gly Tyr Ala Asp Gly Ser Phe Lys
   50 55 60
   Pro Asn Gln Thr Ile Asn Arg Gly Gln Val Val Lys Leu Leu Gly Arg
   65 70 75 80
   Tyr Leu Glu Ala Gln Gly Gln Glu Ile Pro Ala Asp Trp Asn Ser Lys
   85 90 95
   Gln Arg Phe Asn Asp Leu Pro Val Thr Ala Glu Glu Glu Leu Val Lys
   100 105 110
   Tyr Ala Ala Leu Ala Lys Asp Ala Gly Val Phe Asn Gly Ser Asn Gly
   115 120 125
   Asn Leu Asn Ala Ser Gln Thr Met Gln Arg Gln Gln Met Ala Val Val
   130 135 140
   Leu Val Arg Ala Ile Lys Glu Ile Ala Gly Val Asp Leu Val Ala Glu
   145 150 155 160
   Tyr Lys Lys Ala Asn Phe Val Thr Glu Ile Gly Asp Leu Asp Lys Ala
   165 170 175
   Tyr Ser Ala Glu Gln Arg Thr Ala Ile Val Ala Leu Glu Tyr Ala Gly
   180 185 190
   Ile Thr Asn Val Ala His Phe Asn Pro Gly Asn Ser Val Thr Arg Gly
   195 200 205
   Gln Phe Ala Ser Phe Leu Tyr Arg Thr Ile Glu Asn Val Val Asn Asn
   210 215 220
   Pro Glu Ala Gly Val Ala Ala Val Lys Ala Ile Asn Asn Thr Thr Val
   225 230 235 240
   Glu Val Thr Phe Asp Glu Glu Val Asp Asn Val Gln Ala Leu Asn Phe
   245 250 255
   Leu Ile Ser Asp Leu Glu Val Lys Asn Ala Ala Val Lys Leu Thr Asn
   260 265 270
   Lys Lys Val Val Val Leu Thr Thr Ala Ala Gln Thr Ala Asp Lys Glu
   275 280 285
   Tyr Thr Val Ser Leu Gly Glu Glu Lys Ile Gly Thr Phe Lys Gly Ile
   290 295 300
   Ala Ala Val Val Pro Thr Lys Val Asp Leu Val Glu Lys Ser Val Gln
   305 310 315 320
   Gly Lys Leu Gly Gln Gln Val Thr Leu Lys Ala Gln Val Thr Val Ala
   325 330 335
   Glu Gly Gln Thr Lys Ala Gly Ile Pro Val Thr Phe Phe Ile Pro Gly
   340 345 350
   Ser Ala Asn Gly Val Lys Thr Pro Val Thr Val Glu Ala Val Thr Asn
   355 360 365
   Glu Glu Gly Ile Ala Thr Tyr Ser Tyr Thr Arg Tyr Ala Ala Thr Asn
   370 375 380
   Asp Thr Val Thr Val Tyr Ala Asn Gly Asp Arg Ser Lys Phe Ser Thr
   385 390 395 400
   Gly Tyr Val Phe Trp Ala Val Asp Gln Thr Leu Glu Ile Thr Glu Val
   405 410 415
   Thr Thr Gly Ala Thr Ile Asn Asn Gly Ala Asn Lys Thr Tyr Lys Val
   420 425 430
   Thr Tyr Lys His Pro Glu Thr Gly Lys Pro Val Ser Gly Lys Val Leu
   435 440 445
   Asn Val Ser Val Lys Glu Asn Ile Asp Val Thr Val Asp Lys Leu Gln
   450 455 460
   Asn Val Thr Val Asn Gly Ile Ala Val Val Gln Thr Ser Asp Asn Asn
   465 470 475 480
   Thr Arg Ala Ala Gln Ile Thr Thr Asp Ser Lys Gly Glu Ala Thr Phe
   485 490 495
   Thr Val Ser Gly Ser Asn Ala Glu Val Thr Pro Val Val Phe Glu Ala
   500 505 510
   Thr Pro Val Gln Val Thr Asn Thr Asn Gly Ala Val Thr Thr Thr Ser
   515 520 525
   Tyr Thr Gln Lys Tyr Thr Ala Asp Ile Leu Gln Ala Ser Ala Ala Lys
   530 535 540
   Val Thr Phe Gly Ala Val Gln Ala Ala Tyr Thr Leu Glu Val Thr Arg
   545 550 555 560
   Asp Gly Gly Glu Val Ala Ala Thr Gly Val Glu Asn Gly Arg Lys Tyr
   565 570 575
   Asn Leu Val Val Lys Asp Lys Asp Gly Lys Leu Ala Ala Asn Glu Thr
   580 585 590
   Val Asn Val Ala Phe Asn Glu Asp Ile Asp Gly Val Ile Ser Thr Val
   595 600 605
   Thr Ser Ala Glu Phe Val Lys Val Glu Asn Asp Lys Gln Val Arg Tyr
   610 615 620
   Glu Gly Lys Lys Ile Thr Val Lys Thr Asn Ser Lys Gly Glu Ala Ser
   625 630 635 640
   Phe Val Ile Ser Ser Glu Ala Val Asn Thr Tyr Ala Thr Pro Ile Ala
   645 650 655
   Trp Ile Asp Ile Asn Asn Gln Ser Ala Lys Asp Ala Asn Leu Asp Lys
   660 665 670
   Gly Glu Pro Ser Ala Val Ala Pro Ile Ser Tyr Phe Gln Ala Glu Tyr
   675 680 685
   Leu Asp Gly Ser Lys Leu Val Ser Tyr Lys Asp Thr Thr Glu Thr Asp
   690 695 700
   Lys Phe Thr Gly Ser Glu Thr Ala Thr Phe Lys Val Gln Leu Thr Asn
   705 710 715 720
   Gln Ser Gly Lys Val Val Lys Asn Ser Gly Tyr Thr Thr Pro Asn Val
   725 730 735
   Ser Tyr Thr Val Tyr Asn Thr Gly Ala Asn Asn Val Lys Val Asp Gly
   740 745 750
   Val Glu Ile Ala Pro Asn Arg Val His Thr Val Val Ala Lys Asp Gly
   755 760 765
   Val Ile Asn Val Thr Thr Val Asp Asn Lys Ser Ser Ser Val Lys Val
   770 775 780
   Leu Ala Thr Gly Val Ala Lys Gln Thr Asn Gly Asn Lys Glu Phe Ala
   785 790 795 800
   Phe Thr Ser Lys Glu Ala Thr Ala Thr Phe Thr Ala Thr Asn Glu Val
   805 810 815
   Ser Asn Pro Tyr Thr Gly Ala Val Lys His Tyr Asn Thr Asp Lys Lys
   820 825 830
   Thr Ile Thr Phe Asp Asn Lys Asp Ala Ile Lys Tyr Ala Gly Val Lys
   835 840 845
   Gly Lys Thr Tyr Lys Tyr Phe Gly Leu Gly Ser Thr Pro Ile Ala Asn
   850 855 860
   Ala Asp Ala Phe Ile Ala Thr Leu Ser Gly Gln Gly Ala Gly Asn Gln
   865 870 875 880
   Val Thr Val Thr Tyr Lys Glu Glu Asp Asp Val Val Ser Phe Tyr Ile
   885 890 895
   Ile Ser Val Val Asn Gly Gly Ile Gly Asn Pro Thr Thr Asp Pro Ala
   900 905 910
   Leu Ala Asn Gly Val Thr Gly Thr Ile Ala Phe Thr Asn Thr Ser Phe
   915 920 925
   Asn Ala Ser Ala Asn Gln Thr Ile Thr Val Lys Asp Ala Asp Leu Asn
   930 935 940
   Val Asn Ala Thr Val Ala Asp Thr Thr Thr Val Thr Ile Thr Asp Ser
   945 950 955 960
   Ala Lys Thr Thr Phe Asn Ile Thr Leu Thr Glu Thr Gly Val Asn Thr
   965 970 975
   Gly Glu Phe Thr Gly Thr Leu Thr Ser Ala Gln Leu Ala Leu Leu Lys
   980 985 990
   Asp Gly Val Ile Thr Ala Thr Tyr Asn Asp Ala Lys Asn Ala Asn Asn
   995 1000 1005
   Val Ala Ala Thr Ala Thr Ala Thr Ala Thr Leu Asn Thr Thr Val
   1010 1015 1020
   Gly Ala Val Asn Phe Ala Ala Lys Ala Thr Thr Glu Tyr Ser Glu
   1025 1030 1035
   Gly Val Gly Thr Ala Ala Lys Val Glu Gly Ser Thr Val Leu Thr
   1040 1045 1050
   Ser Lys Val Asp Thr Gly Asp Leu Val Leu Val Val Asp Gly Val
   1055 1060 1065
   Gln Phe Thr Thr Val Val Thr Ala Ile Asp Pro Gly Thr Ser Ala
   1070 1075 1080
   Thr Ala Ser Leu Thr Ala Val Val Ala Glu Ile Asn Ala Ala Ala
   1085 1090 1095
   Lys Lys Val Gly Phe Thr Ala Asp Val Ala Thr Val Asp Ala Asp
   1100 1105 1110
   Lys Ile Val Leu Thr Ser Pro Thr Lys Gly Thr Ser Ser Ser Val
   1115 1120 1125
   Glu Val Lys Asp Leu Gly Thr Thr Thr Gly Leu Gly Leu Thr Lys
   1130 1135 1140
   Ala Pro Glu Val Lys Gly Thr Ala Gly Ala Ala Val Ala Thr Gln
   1145 1150 1155
   Trp Lys Phe Thr Val Thr Thr Thr Pro Ala Val Gly Glu Thr Ile
   1160 1165 1170
   Thr Val Lys Val Gly Thr Lys Glu Ala Ser His Lys Val Val Ala
   1175 1180 1185
   Gly Asn Asp Leu Ala Ala Ile Ala Thr Ala Leu Asn Thr Ala Ile
   1190 1195 1200
   Gly Ala Asp Tyr Asn Ile Ala Leu Val Thr Gly Ser Asn Val Ile
   1205 1210 1215
   Thr Val Thr Gln Ala Thr Pro Ala Lys Thr Thr Asp Glu Leu Ser
   1220 1225 1230
   Val Thr Val Thr Lys
   1235
<210> 7
   <211> 3303
   <212> DNA
   <213> Lysinibacillus sphaericus
<220>
   <221> CDS
   <222> (1)..(3303)
<400> 7
   atg gct aac caa cca aag aaa tac aaa aaa ttc gta gcg acg gca gca       48
   Met Ala Asn Gln Pro Lys Lys Tyr Lys Lys Phe Val Ala Thr Ala Ala
   1               5                   10                  15
   acg gca aca tta gta gca tct gct atc gta cca gtg gtt tct gcc gca       96
   Thr Ala Thr Leu Val Ala Ser Ala Ile Val Pro Val Val Ser Ala Ala
               20                  25                  30
   gga ttt tca gat gta gca ggt aac gac cac gaa gta gca atc aac gca      144
   Gly Phe Ser Asp Val Ala Gly Asn Asp His Glu Val Ala Ile Asn Ala
           35                  40                  45
   ctt gct gat gca ggt atc atc aat gga tac gct gac ggc aca ttc aaa      192
   Leu Ala Asp Ala Gly Ile Ile Asn Gly Tyr Ala Asp Gly Thr Phe Lys
       50                  55                  60
   cca aac caa aca att aac cgc ggt caa gtg gtt aaa tta tta ggt cgt      240
   Pro Asn Gln Thr Ile Asn Arg Gly Gln Val Val Lys Leu Leu Gly Arg
   65                  70                  75                  80
   tat tta gaa gca caa ggc caa gaa att cca gct gac tgg aat tca aaa        288
   Tyr Leu Glu Ala Gln Gly Gln Glu Ile Pro Ala Asp Trp Asn Ser Lys
                   85                  90                  95
   caa cgc ttc aac gat cta cca gta aca gct gaa gca gaa tta gta aaa        336
   Gln Arg Phe Asn Asp Leu Pro Val Thr Ala Glu Ala Glu Leu Val Lys
               100                 105                 110
   tat gct gca cta gca aaa gat gca ggc gta ttc aac ggt tca aac ggc        384
   Tyr Ala Ala Leu Ala Lys Asp Ala Gly Val Phe Asn Gly Ser Asn Gly
           115                 120                 125
   aac tta aac gct tca caa aca atg caa cgt caa caa atg gca gta gtt        432
   Asn Leu Asn Ala Ser Gln Thr Met Gln Arg Gln Gln Met Ala Val Val
       130                 135                 140
   tta gta cgt gct att aaa gaa atc gca ggc gta gac tta gta gct gag        480
   Leu Val Arg Ala Ile Lys Glu Ile Ala Gly Val Asp Leu Val Ala Glu
   145                 150                 155                 160
   tac aaa aaa gcg aac ttc gta aca gaa att ggt gac tta gac aaa gct        528
   Tyr Lys Lys Ala Asn Phe Val Thr Glu Ile Gly Asp Leu Asp Lys Ala
                   165                 170                 175
   tac tct gca gaa caa cgc aca gcg atc gtt gct tta gaa tat gcg ggt        576
   Tyr Ser Ala Glu Gln Arg Thr Ala Ile Val Ala Leu Glu Tyr Ala Gly
               180                 185                 190
   att aca aat gtt gct cac ttc aac cca ggt aac agc gta aca cgc gga        624
   Ile Thr Asn Val Ala His Phe Asn Pro Gly Asn Ser Val Thr Arg Gly
           195                 200                 205
   caa ttc gct tca ttc tta tac cgc aca atc gaa aac gtt gta aac aac        672
   Gln Phe Ala Ser Phe Leu Tyr Arg Thr Ile Glu Asn Val Val Asn Asn
       210                 215                 220
   cca gaa gct ggt gtt gcg gca gta aaa gct atc aac aac aca act gtt        720
   Pro Glu Ala Gly Val Ala Ala Val Lys Ala Ile Asn Asn Thr Thr Val
   225                 230                 235                 240
   gaa gta aca ttc gac gaa gaa gta gac aac gta caa gct ctt aac ttc        768
   Glu Val Thr Phe Asp Glu Glu Val Asp Asn Val Gln Ala Leu Asn Phe
                   245                 250                 255
   tta atc tct gat ctt gaa gtg aaa aac gca gct gtt aaa tta aca aac        816
   Leu Ile Ser Asp Leu Glu Val Lys Asn Ala Ala Val Lys Leu Thr Asn
               260                 265                 270
   aaa aaa gtt gtt gta tta aca act gct gct caa aca gct gac aaa gag        864
   Lys Lys Val Val Val Leu Thr Thr Ala Ala Gln Thr Ala Asp Lys Glu
           275                 280                 285
   tac act gta tct ctt ggc gaa gaa aaa atc ggt aca ttc aaa ggt atc        912
   Tyr Thr Val Ser Leu Gly Glu Glu Lys Ile Gly Thr Phe Lys Gly Ile
       290                 295                 300
   gca gct gta gtt cct act aaa gta gat tta gta gaa aaa tca gtt caa        960
   Ala Ala Val Val Pro Thr Lys Val Asp Leu Val Glu Lys Ser Val Gln
   305                 310                 315                 320
   ggt aaa ctt ggc caa caa gta aca ctt aaa gca caa gta act gtt gct       1008
   Gly Lys Leu Gly Gln Gln Val Thr Leu Lys Ala Gln Val Thr Val Ala
                   325                 330                 335
   gaa ggt caa aca aaa gct ggt att cca gta act ttc ttc atc cca ggt       1056
   Glu Gly Gln Thr Lys Ala Gly Ile Pro Val Thr Phe Phe Ile Pro Gly
               340                 345                 350
   tct gct aat ggc gtg aaa act cca gta aca gtt gaa gct gta aca aac    1104
   Ser Ala Asn Gly Val Lys Thr Pro Val Thr Val Glu Ala Val Thr Asn
           355                 360                 365
   gaa gaa ggt atc gca act tac tct tac act cgt tat gct gca aca aac    1152
   Glu Glu Gly Ile Ala Thr Tyr Ser Tyr Thr Arg Tyr Ala Ala Thr Asn
       370                 375                 380
   gac act gta act gta tat gca aac ggt gac cgt tct aaa ttc tca act    1200
   Asp Thr Val Thr Val Tyr Ala Asn Gly Asp Arg Ser Lys Phe Ser Thr
   385                 390                 395                 400
   ggt tat gta ttc tgg gca gtt gac caa act tta gaa atc act gaa gta    1248
   Gly Tyr Val Phe Trp Ala Val Asp Gln Thr Leu Glu Ile Thr Glu Val
                   405                 410                 415
   aca act ggt gct act atc aac aac ggc gct aac aaa act tac aaa gtt    1296
   Thr Thr Gly Ala Thr Ile Asn Asn Gly Ala Asn Lys Thr Tyr Lys Val
               420                 425                 430
   act tac aaa cac cca gaa act ggt aaa cca gta tca ggt aaa gta ctg    1344
   Thr Tyr Lys His Pro Glu Thr Gly Lys Pro Val Ser Gly Lys Val Leu
           435                 440                 445
   aac gtt tca gta aaa gaa aac att gac gta act gtt gat aaa tta caa    1392
   Asn Val Ser Val Lys Glu Asn Ile Asp Val Thr Val Asp Lys Leu Gln
       450                 455                 460
   aac gtt acg gtg aac ggg gta gcg gta gtt caa acg agt aca aac aac    1440
   Asn Val Thr Val Asn Gly Val Ala Val Val Gln Thr Ser Thr Asn Asn
   465                 470                 475                 480
   acg aga gcc gca caa atc aca aca gat tct aga ggt gaa gca aca ttt    1488
   Thr Arg Ala Ala Gln Ile Thr Thr Asp Ser Arg Gly Glu Ala Thr Phe
                   485                 490                 495
   aca gtg tca ggt tcg aac gct gta gtt aca cca gtc gta ttt gaa gct    1536
   Thr Val Ser Gly Ser Asn Ala Val Val Thr Pro Val Val Phe Glu Ala
               500                 505                 510
   act gca acc aat gta gct aac acg tat tct caa aca tac aca tca gat    1584
   Thr Ala Thr Asn Val Ala Asn Thr Tyr Ser Gln Thr Tyr Thr Ser Asp
           515                 520                 525
   gta tta caa gca tct gca gcc aaa gta aca ttt ggt gct att caa gtt    1632
   Val Leu Gln Ala Ser Ala Ala Lys Val Thr Phe Gly Ala Ile Gln Val
       530                 535                 540
   gct tat acg ctt gaa gta acg cgt gat ggt ggt gaa gtg gca gct act    1680
   Ala Tyr Thr Leu Glu Val Thr Arg Asp Gly Gly Glu Val Ala Ala Thr
   545                 550                 555                 560
   ggt gta aca aac ggt cgc aaa tat aac atc gtt gtg aaa gat aag gat    1728
   Gly Val Thr Asn Gly Arg Lys Tyr Asn Ile Val Val Lys Asp Lys Asp
                   565                 570                 575
   ggt aaa gta gca ccg aac gaa atc gtt aac gta gca ttt aac gaa gat    1776
   Gly Lys Val Ala Pro Asn Glu Ile Val Asn Val Ala Phe Asn Glu Asp
               580                 585                 590
   att gat ggc gta att tct aca gtt act tct gca caa ttc gtg aaa gta    1824
   Ile Asp Gly Val Ile Ser Thr Val Thr Ser Ala Gln Phe Val Lys Val
           595                 600                 605
   gaa gac ggt aaa caa gtg ggt tac gaa ggt aaa aaa atc act gtt aga    1872
   Glu Asp Gly Lys Gln Val Gly Tyr Glu Gly Lys Lys Ile Thr Val Arg
       610                 615                 620
   aca aac gct aaa ggt gaa gca agc ttc gtc atc ggc agt gat act gta      1920
   Thr Asn Ala Lys Gly Glu Ala Ser Phe Val Ile Gly Ser Asp Thr Val
   625                 630                 635                 640
   aat tct tac gct aca cca gtc gtt tgg atc gat att aac aac caa agc      1968
   Asn Ser Tyr Ala Thr Pro Val Val Trp Ile Asp Ile Asn Asn Gln Ser
                   645                 650                 655
   ggt aaa gat gca aac tta gac aaa ggt gaa cct tca gca gta gca cca      2016
   Gly Lys Asp Ala Asn Leu Asp Lys Gly Glu Pro Ser Ala Val Ala Pro
               660                 665                 670
   atc tca tac ttc caa gct cca tat tta gac ggt tca aaa tta gta tct      2064
   Ile Ser Tyr Phe Gln Ala Pro Tyr Leu Asp Gly Ser Lys Leu Val Ser
           675                 680                 685
   tat aaa ggt aca act gaa acg gat aaa ttt gca ggt ggc gaa gta gca      2112
   Tyr Lys Gly Thr Thr Glu Thr Asp Lys Phe Ala Gly Gly Glu Val Ala
       690                 695                 700
   aca ttt aaa gta caa ctt aca aac caa agt ggt aaa gtc gta cca aat      2160
   Thr Phe Lys Val Gln Leu Thr Asn Gln Ser Gly Lys Val Val Pro Asn
   705                 710                 715                 720
   tct ggt tac aca acg aag gat gtt acg tac acg gtt tac aat acc ggc      2208
   Ser Gly Tyr Thr Thr Lys Asp Val Thr Tyr Thr Val Tyr Asn Thr Gly
                   725                 730                 735
   gca aac aat ata aaa gtt ggt act gta gaa atc gca cca aac cgc gtg      2256
   Ala Asn Asn Ile Lys Val Gly Thr Val Glu Ile Ala Pro Asn Arg Val
               740                 745                 750
   cat aca gta gtt gcg cca agt ggt gaa att act gtt act act gta gat      2304
   His Thr Val Val Ala Pro Ser Gly Glu Ile Thr Val Thr Thr Val Asp
           755                 760                 765
   ggt aaa act tct tct gta cga gtt tta gct aca ggt gta gcg aaa gag      2352
   Gly Lys Thr Ser Ser Val Arg Val Leu Ala Thr Gly Val Ala Lys Glu
       770                 775                 780
   gtt aac ggg aat aaa gaa ttt gca ttt act tca aaa gaa gca act gca      2400
   Val Asn Gly Asn Lys Glu Phe Ala Phe Thr Ser Lys Glu Ala Thr Ala
   785                 790                 795                 800
   aca ttc act tct act tca gaa atc cct aac cta ttt act ggt gat att      2448
   Thr Phe Thr Ser Thr Ser Glu Ile Pro Asn Leu Phe Thr Gly Asp Ile
                   805                 810                 815
   cat tca ttc gat aca gac aaa gaa act gtc gta ttt act gat ggt tcc      2496
   His Ser Phe Asp Thr Asp Lys Glu Thr Val Val Phe Thr Asp Gly Ser
               820                 825                 830
   gtt cta aaa aat gcg gtg aaa tat gct ggt gaa acg ggc aaa aca tat      2544
   Val Leu Lys Asn Ala Val Lys Tyr Ala Gly Glu Thr Gly Lys Thr Tyr
           835                 840                 845
   gta tat aga aat gta aat ggt gca cca ctt tct aaa gac cag ttt gta      2592
   Val Tyr Arg Asn Val Asn Gly Ala Pro Leu Ser Lys Asp Gln Phe Val
       850                 855                 860
   aat att ctt aaa ggt gcc act tca ggg gta aaa gta aca tat gaa gtt      2640
   Asn Ile Leu Lys Gly Ala Thr Ser Gly Val Lys Val Thr Tyr Glu Val
   865                 870                 875                 880
   aaa gat aat act gtt aca ttc tat gtt gtt tct gtt aac gct ggg gga      2688
   Lys Asp Asn Thr Val Thr Phe Tyr Val Val Ser Val Asn Ala Gly Gly
                   885                 890                 895
   act aag ccg gtt gat att att gga gat aaa gca tct gac gct act gct      2736
   Thr Lys Pro Val Asp Ile Ile Gly Asp Lys Ala Ser Asp Ala Thr Ala
               900                 905                 910
   gtt aca gct gca aaa gct aaa gtt aca gta aca cct aat att gct aca      2784
   Val Thr Ala Ala Lys Ala Lys Val Thr Val Thr Pro Asn Ile Ala Thr
           915                 920                 925
   ctt tca act gat acg gca gaa aat aaa cgt tta gct gta caa tca aat      2832
   Leu Ser Thr Asp Thr Ala Glu Asn Lys Arg Leu Ala Val Gln Ser Asn
       930                 935                 940
   gtt gta act gaa att aat gac tca tca gta act gta agt gta gta gaa      2880
   Val Val Thr Glu Ile Asn Asp Ser Ser Val Thr Val Ser Val Val Glu
   945                 950                 955                 960
   act tct aca gca gga act tat act gtt acg tta act aaa ggc gca agc      2928
   Thr Ser Thr Ala Gly Thr Tyr Thr Val Thr Leu Thr Lys Gly Ala Ser
                   965                 970                 975
   act gct aca gta aat gtt aca cca aaa ttt ggt caa aaa gta act gct      2976
   Thr Ala Thr Val Asn Val Thr Pro Lys Phe Gly Gln Lys Val Thr Ala
               980                 985                 990
   gaa tct att act tct tca ctt gca  gtg aat aca aat gga  aaa gat tta    3024
   Glu Ser Ile Thr Ser Ser Leu Ala  Val Asn Thr Asn Gly  Lys Asp Leu
           995                 1000                 1005
   acc ctt  aat att ggg tct gct  ctt gca ggt ttc aca  atc gcg aat       3069
   Thr Leu  Asn Ile Gly Ser Ala  Leu Ala Gly Phe Thr  Ile Ala Asn
       1010                 1015                 1020
   gta aat  cca gat gct gat aat  gca tta gac att act  act tct ttt       3114
   Val Asn  Pro Asp Ala Asp Asn  Ala Leu Asp Ile Thr  Thr Ser Phe
       1025                 1030                 1035
   aat aat  aat gtg att act ata  gct aaa gca cct tct  gct tct att       3159
   Asn Asn  Asn Val Ile Thr Ile  Ala Lys Ala Pro Ser  Ala Ser Ile
       1040                 1045                 1050
   gaa gct  aca caa tta atc gag  aag act tta aaa gtt  tat ttc gac       3204
   Glu Ala  Thr Gln Leu Ile Glu  Lys Thr Leu Lys Val  Tyr Phe Asp
       1055                 1060                 1065
   gct caa  gct act ggt tat gct  aaa gaa agc ttt gta  gca act att       3249
   Ala Gln  Ala Thr Gly Tyr Ala  Lys Glu Ser Phe Val  Ala Thr Ile
       1070                 1075                 1080
   aca att  gat gat gta acg aac  cgt act ttg gct att  aca gta aag       3294
   Thr Ile  Asp Asp Val Thr Asn  Arg Thr Leu Ala Ile  Thr Val Lys
       1085                 1090                 1095
   cct act  cca                                                         3303
   Pro Thr  Pro
       1100
<210> 8
   <211> 1101
   <212> PRT
   <213> Lysinibacillus sphaericus
<400> 8
   Met Ala Asn Gln Pro Lys Lys Tyr Lys Lys Phe Val Ala Thr Ala Ala
   1               5                   10                  15
   Thr Ala Thr Leu Val Ala Ser Ala Ile Val Pro Val Val Ser Ala Ala
               20                  25                  30
   Gly Phe Ser Asp Val Ala Gly Asn Asp His Glu Val Ala Ile Asn Ala
           35                  40                  45
   Leu Ala Asp Ala Gly Ile Ile Asn Gly Tyr Ala Asp Gly Thr Phe Lys
       50                  55                  60
   Pro Asn Gln Thr Ile Asn Arg Gly Gln Val Val Lys Leu Leu Gly Arg
   65                  70                  75                  80
   Tyr Leu Glu Ala Gln Gly Gln Glu Ile Pro Ala Asp Trp Asn Ser Lys
                   85                  90                  95
   Gln Arg Phe Asn Asp Leu Pro Val Thr Ala Glu Ala Glu Leu Val Lys
               100                 105                 110
   Tyr Ala Ala Leu Ala Lys Asp Ala Gly Val Phe Asn Gly Ser Asn Gly
           115                 120                 125
   Asn Leu Asn Ala Ser Gln Thr Met Gln Arg Gln Gln Met Ala Val Val
       130                 135                 140
   Leu Val Arg Ala Ile Lys Glu Ile Ala Gly Val Asp Leu Val Ala Glu
   145                 150                 155                 160
   Tyr Lys Lys Ala Asn Phe Val Thr Glu Ile Gly Asp Leu Asp Lys Ala
                   165                 170                 175
   Tyr Ser Ala Glu Gln Arg Thr Ala Ile Val Ala Leu Glu Tyr Ala Gly
               180                 185                 190
   Ile Thr Asn Val Ala His Phe Asn Pro Gly Asn Ser Val Thr Arg Gly
           195                 200                 205
   Gln Phe Ala Ser Phe Leu Tyr Arg Thr Ile Glu Asn Val Val Asn Asn
       210                 215                 220
   Pro Glu Ala Gly Val Ala Ala Val Lys Ala Ile Asn Asn Thr Thr Val
   225                 230                 235                 240
   Glu Val Thr Phe Asp Glu Glu Val Asp Asn Val Gln Ala Leu Asn Phe
                   245                 250                 255
   Leu Ile Ser Asp Leu Glu Val Lys Asn Ala Ala Val Lys Leu Thr Asn
               260                 265                 270
   Lys Lys Val Val Val Leu Thr Thr Ala Ala Gln Thr Ala Asp Lys Glu
           275                 280                 285
   Tyr Thr Val Ser Leu Gly Glu Glu Lys Ile Gly Thr Phe Lys Gly Ile
       290                 295                 300
   Ala Ala Val Val Pro Thr Lys Val Asp Leu Val Glu Lys Ser Val Gln
   305 310 315 320
   Gly Lys Leu Gly Gln Gln Val Thr Leu Lys Ala Gln Val Thr Val Ala
   325 330 335
   Glu Gly Gln Thr Lys Ala Gly Ile Pro Val Thr Phe Phe Ile Pro Gly
   340 345 350
   Ser Ala Asn Gly Val Lys Thr Pro Val Thr Val Glu Ala Val Thr Asn
   355 360 365
   Glu Glu Gly Ile Ala Thr Tyr Ser Tyr Thr Arg Tyr Ala Ala Thr Asn
   370 375 380
   Asp Thr Val Thr Val Tyr Ala Asn Gly Asp Arg Ser Lys Phe Ser Thr
   385 390 395 400
   Gly Tyr Val Phe Trp Ala Val Asp Gln Thr Leu Glu Ile Thr Glu Val
   405 410 415
   Thr Thr Gly Ala Thr Ile Asn Asn Gly Ala Asn Lys Thr Tyr Lys Val
   420 425 430
   Thr Tyr Lys His Pro Glu Thr Gly Lys Pro Val Ser Gly Lys Val Leu
   435 440 445
   Asn Val Ser Val Lys Glu Asn Ile Asp Val Thr Val Asp Lys Leu Gln
   450 455 460
   Asn Val Thr Val Asn Gly Val Ala Val Val Gln Thr Ser Thr Asn Asn
   465 470 475 480
   Thr Arg Ala Ala Gln Ile Thr Thr Asp Ser Arg Gly Glu Ala Thr Phe
   485 490 495
   Thr Val Ser Gly Ser Asn Ala Val Val Thr Pro Val Val Phe Glu Ala
   500 505 510
   Thr Ala Thr Asn Val Ala Asn Thr Tyr Ser Gln Thr Tyr Thr Ser Asp
   515 520 525
   Val Leu Gln Ala Ser Ala Ala Lys Val Thr Phe Gly Ala Ile Gln Val
   530 535 540
   Ala Tyr Thr Leu Glu Val Thr Arg Asp Gly Gly Glu Val Ala Ala Thr
   545 550 555 560
   Gly Val Thr Asn Gly Arg Lys Tyr Asn Ile Val Val Lys Asp Lys Asp
   565 570 575
   Gly Lys Val Ala Pro Asn Glu Ile Val Asn Val Ala Phe Asn Glu Asp
   580 585 590
   Ile Asp Gly Val Ile Ser Thr Val Thr Ser Ala Gln Phe Val Lys Val
   595 600 605
   Glu Asp Gly Lys Gln Val Gly Tyr Glu Gly Lys Lys Ile Thr Val Arg
   610 615 620
   Thr Asn Ala Lys Gly Glu Ala Ser Phe Val Ile Gly Ser Asp Thr Val
   625 630 635 640
   Asn Ser Tyr Ala Thr Pro Val Val Trp Ile Asp Ile Asn Asn Gln Ser
   645 650 655
   Gly Lys Asp Ala Asn Leu Asp Lys Gly Glu Pro Ser Ala Val Ala Pro
   660 665 670
   Ile Ser Tyr Phe Gln Ala Pro Tyr Leu Asp Gly Ser Lys Leu Val Ser
   675 680 685
   Tyr Lys Gly Thr Thr Glu Thr Asp Lys Phe Ala Gly Gly Glu Val Ala
   690 695 700
   Thr Phe Lys Val Gln Leu Thr Asn Gln Ser Gly Lys Val Val Pro Asn
   705 710 715 720
   Ser Gly Tyr Thr Thr Lys Asp Val Thr Tyr Thr Val Tyr Asn Thr Gly
   725 730 735
   Ala Asn Asn Ile Lys Val Gly Thr Val Glu Ile Ala Pro Asn Arg Val
   740 745 750
   His Thr Val Val Ala Pro Ser Gly Glu Ile Thr Val Thr Thr Val Asp
   755 760 765
   Gly Lys Thr Ser Ser Val Arg Val Leu Ala Thr Gly Val Ala Lys Glu
   770 775 780
   Val Asn Gly Asn Lys Glu Phe Ala Phe Thr Ser Lys Glu Ala Thr Ala
   785 790 795 800
   Thr Phe Thr Ser Thr Ser Glu Ile Pro Asn Leu Phe Thr Gly Asp Ile
   805 810 815
   His Ser Phe Asp Thr Asp Lys Glu Thr Val Val Phe Thr Asp Gly Ser
   820 825 830
   Val Leu Lys Asn Ala Val Lys Tyr Ala Gly Glu Thr Gly Lys Thr Tyr
   835 840 845
   Val Tyr Arg Asn Val Asn Gly Ala Pro Leu Ser Lys Asp Gln Phe Val
   850 855 860
   Asn Ile Leu Lys Gly Ala Thr Ser Gly Val Lys Val Thr Tyr Glu Val
   865 870 875 880
   Lys Asp Asn Thr Val Thr Phe Tyr Val Val Ser Val Asn Ala Gly Gly
   885 890 895
   Thr Lys Pro Val Asp Ile Ile Gly Asp Lys Ala Ser Asp Ala Thr Ala
   900 905 910
   Val Thr Ala Ala Lys Ala Lys Val Thr Val Thr Pro Asn Ile Ala Thr
   915 920 925
   Leu Ser Thr Asp Thr Ala Glu Asn Lys Arg Leu Ala Val Gln Ser Asn
   930 935 940
   Val Val Thr Glu Ile Asn Asp Ser Ser Val Thr Val Ser Val Val Glu
   945 950 955 960
   Thr Ser Thr Ala Gly Thr Tyr Thr Val Thr Leu Thr Lys Gly Ala Ser
   965 970 975
   Thr Ala Thr Val Asn Val Thr Pro Lys Phe Gly Gln Lys Val Thr Ala
   980 985 990
   Glu Ser Ile Thr Ser Ser Leu Ala Val Asn Thr Asn Gly Lys Asp Leu
   995 1000 1005
   Thr Leu Asn Ile Gly Ser Ala Leu Ala Gly Phe Thr Ile Ala Asn
   1010 1015 1020
   Val Asn Pro Asp Ala Asp Asn Ala Leu Asp Ile Thr Thr Ser Phe
   1025 1030 1035
   Asn Asn Asn Val Ile Thr Ile Ala Lys Ala Pro Ser Ala Ser Ile
   1040 1045 1050
   Glu Ala Thr Gln Leu Ile Glu Lys Thr Leu Lys Val Tyr Phe Asp
   1055 1060 1065
   Ala Gln Ala Thr Gly Tyr Ala Lys Glu Ser Phe Val Ala Thr Ile
   1070 1075 1080
   Thr Ile Asp Asp Val Thr Asn Arg Thr Leu Ala Ile Thr Val Lys
   1085 1090 1095
   Pro Thr Pro
   1100
<210> 9
   <211> 277
   <212> PRT
   <213> Lysinibacillus sphaericus
<400> 9
   Thr Val Val Phe Thr Asp Gly Ser Val Leu Lys Asn Ala Val Lys Tyr
   1               5                   10                  15
   Ala Gly Glu Thr Gly Lys Thr Tyr Val Tyr Arg Asn Val Asn Gly Ala
               20                  25                  30
   Pro Leu Ser Lys Asp Gln Phe Val Asn Ile Leu Lys Gly Ala Thr Ser
           35                  40                  45
   Gly Val Lys Val Thr Tyr Glu Val Lys Asp Asn Thr Val Thr Phe Tyr
       50                  55                  60
   Val Val Ser Val Asn Ala Gly Gly Thr Lys Pro Val Asp Ile Ile Gly
   65                  70                  75                  80
   Asp Lys Ala Ser Asp Ala Thr Ala Val Thr Ala Ala Lys Ala Lys Val
                   85                  90                  95
   Thr Val Thr Pro Asn Ile Ala Thr Leu Ser Thr Asp Thr Ala Glu Asn
               100                 105                 110
   Lys Arg Leu Ala Val Gln Ser Asn Val Val Thr Glu Ile Asn Asp Ser
           115                 120                 125
   Ser Val Thr Val Ser Val Val Glu Thr Ser Thr Ala Gly Thr Tyr Thr
       130                 135                 140
   Val Thr Leu Thr Lys Gly Ala Ser Thr Ala Thr Val Asn Val Thr Pro
   145                 150                 155                 160
   Lys Phe Gly Gln Lys Val Thr Ala Glu Ser Ile Thr Ser Ser Leu Ala
                   165                 170                 175
   Val Asn Thr Asn Gly Lys Asp Leu Thr Leu Asn Ile Gly Ser Ala Leu
               180                 185                 190
   Ala Gly Phe Thr Ile Ala Asn Val Asn Pro Asp Ala Asp Asn Ala Leu
           195                 200                 205
   Asp Ile Thr Thr Ser Phe Asn Asn Asn Val Ile Thr Ile Ala Lys Ala
       210                 215                 220
   Pro Ser Ala Ser Ile Glu Ala Thr Gln Leu Ile Glu Lys Thr Leu Lys
   225                 230                 235                 240
   Val Tyr Phe Asp Ala Gln Ala Thr Gly Tyr Ala Lys Glu Ser Phe Val
                   245                 250                 255
   Ala Thr Ile Thr Ile Asp Asp Val Thr Asn Arg Thr Leu Ala Ile Thr
               260                 265                 270
   Val Lys Pro Thr Pro
           275
<210> 10
   <211> 831
   <212> DNA
   <213> Lysinibacillus sphaericus
<400> 10
   actgtcgtat ttactgatgg ttccgttcta aaaaatgcgg tgaaatatgc tggtgaaacg        60
   ggcaaaacat atgtatatag aaatgtaaat ggtgcaccac tttctaaaga ccagtttgta       120
   aatattctta aaggtgccac ttcaggggta aaagtaacat atgaagttaa agataatact       180
   gttacattct atgttgtttc tgttaacgct gggggaacta agccggttga tattattgga       240
   gataaagcat ctgacgctac tgctgttaca gctgcaaaag ctaaagttac agtaacacct       300
   aatattgcta cactttcaac tgatacggca gaaaataaac gtttagctgt acaatcaaat       360
   gttgtaactg aaattaatga ctcatcagta actgtaagtg tagtagaaac ttctacagca       420
   ggaacttata ctgttacgtt aactaaaggc gcaagcactg ctacagtaaa tgttacacca       480
   aaatttggtc aaaaagtaac tgctgaatct attacttctt cacttgcagt gaatacaaat       540
   ggaaaagatt taacccttaa tattgggtct gctcttgcag gtttcacaat cgcgaatgta       600
   aatccagatg ctgataatgc attagacatt actacttctt ttaataataa tgtgattact       660
   atagctaaag caccttctgc ttctattgaa gctacacaat taatcgagaa gactttaaaa       720
   gtttatttcg acgctcaagc tactggttat gctaaagaaa gctttgtagc aactattaca       780
   attgatgatg taacgaaccg tactttggct attacagtaa agcctactcc a                831
<210> 11
   <211> 36
   <212> DNA
   <213> Artificial sequence
<220>
   <223> Oligo
<400> 11
   gacgacgaca agatgatcaa caacacaact gttgaa 36
<210> 12
   <211> 37
   <212> DNA
   <213> Artificial sequence
<220>
   <223> oligo
<400> 12
   gaggagaagc ccggttggag ttggctttac tgtaata 37

## Beschreibung

Die vorliegende Erfindung betrifft ein *E*. *coli*-Sekretionssystem auf Basis einer S-Layer-Gensequenz, deren Expressionsprodukt die Sekretion von rekombinanten Proteinen aus *E*. *coli*-Zellen vermittelt. Das Sekretionssystem eignet sich für die Anwendung in der Molekularbiologie und in der Biotechnologie.

*E. coli* ist der am weitesten verbreitete Wirt für die Herstellung von rekombinanten Proteinen. Häufig treten aber dabei Probleme auf, wie z. B. eine toxische Wirkung des Genproduktes bei Überproduktion oder inkorrekt gefaltete Proteine und daraus folgend die Bildung von sogenannten "inclusion bodies", Einschlusskörperchen im Cytoplasma, die aus Ansammlungen von fehlerhaft gefalteten Proteinen bestehen.

Eine Lösung für diese Probleme ist die Sekretion der rekombinanten Proteine in den periplasmatischen Raum oder in das Kulturmedium. Für die Ermöglichung einer effizienten Sekretion werden unterschiedliche Strategien genutzt. So können die in *E. coli* natürlicherweise vorhandenen Sekretionssysteme (Typ I, Typ II, Typ III) für einen Export der rekombinanten Proteine genutzt werden. Die zu sezernierenden Proteine tragen eine kurze Signalsequenz von 15-30 Aminosäuren (z.B. von OmpA, PelB, PhoA, Endoxylanase), dieeinen Export in den extrazellulären Raum ermöglicht. Wird eine solche Signalsequenz mit rekombinanten Proteinen fusioniert, kann ein Export dieser Proteine ermöglicht werden. Die Effizienz der Proteinsekretion wird dabei bestimmt durch den Wirtsstamm, die Signalsequenz und das Protein. Welche Kombination gut funktioniert, ist nicht vorhersagbar und muss individuell experimentell ermittelt werden. Eine Liste von bekannten und für die Sekretion von rekombinanten Proteinen verwendeten Signalsequenzen ist beispielsweise in folgendem Review-Artikel gegeben: Choi, J.H. & Lee, S. Y., 2004, Appl. Microbiol. Biotechnol. 64, pp. 625-635.

WO 00/49163 A1 offenbart, dass der C-Terminus des S-Layer-Proteins *Caulobacter crescentus* eine Signalsequenz für die Sekretion über einen Typ I-Transporter in *Caulobacter* enthält. Diese Sequenz wird nicht abgespalten. Die Signalsequenz wurde mit anderen Proteinen und Peptiden fusioniert, in *Caulobacter* exprimiert und eine Sekretion nachgewiesen.

In den Stämmen *Lysinibacillus sphaericus* JG-A12 und *Lysinibacillus sphaericus* NCTC9602 wurden S-Layer-Proteine mit N-terminalen Signalsequenzen beschrieben (Pollmann, K. et al. (2005), Microbiology, 151, pp. 2961-2973). Die beschriebenen Signalsequenzen entsprechen typischen bekannten S-Layer-Signalsequenzen für gram-positive Bakterien mit Schnittstellen zwischen den Aminosäuren 31 und 32.

US2002/048816 A1 beschreibt ein Expressionssystem, bei dem das S-Layer-Protein des Stammes *Bacillus sphaericus* P-1 C- oder N-terminal mit verschiedenen Polypeptiden fusioniert wird.

WO97/28263 A1 beschreibt ein Verfahren zur Herstellung von rekombinanten S-Layer-Proteinen in gram-negativen Wirtszellen. Für das Verfahren wird die für das S-Layer-Protein codierende Sequenz mit einer für ein Signalpeptid aus einem gram-positiven Bakterium operativ verknüpft. Die Experimente zeigten, dass die Proteine im Cytosol von *E*. *coli*-Zellen akkumulieren. Jedoch wurde eine Präsenz im Kulturüberstand nicht nachgewiesen. Das beschriebene Signalpeptid ist somit funktionell nicht aktiv. Auch Bowditch et al. (Bowditch, D. et al. (1989), J. Bacteriol. 171, pp. 4178-4188) beschreibt die Klonierung des S-Layer-Gens sowie eines kryptischen Gens mit Signalsequenz von *Bacillus sphaericus* 2362 in *E*. *coli.* Es konnte eine Expression nachgewiesen werden, jedoch keine Sekretion in das Medium.

Es gibt zwar viele Beispiele für eine erfolgreiche Sekretion von rekombinanten Proteinen in das Periplasma, aber immer noch treten sehr häufig Probleme auf, für deren Lösung auf unterschiedliche Strategien zurückgegriffen wird. Die Bildung von inclusion bodies im Periplasma kann durch die Koexpression von Chaperonen im Periplasma verhindert werden. Auftretende Probleme bei der korrekten Faltung der rekombinanten Proteine im Periplasma können auch durch die Verwendung des TAT-Systems umgangen werden, das ein Transportsystem für bereits gefaltete Proteine darstellt. Degradation von rekombinanten Proteinen durch Proteasen kann vermieden werden, indem Protease-negative Stämme verwendet werden (Choi, J.H. & Lee, S. Y., 2004, Appl. Microbiol. Biotechnol. 64, pp. 625-635).

Dies verhindert eine Akkumulation der Proteine in der Zelle und damit eine toxische Wirkung und unterstützt die korrekte Faltung der Proteine. Zudem wird durch die Sekretion eine Aufreinigung der Proteine erleichtert, da sie nicht von den cytosolischen Proteinen separiert werden müssen und den Hauptanteil der extrazellulären bzw. periplasmatischen Proteinfraktion bilden. Außerdem ist im Periplasma die Proteaseaktivität wesentlich geringer im Vergleich zum Cytoplasma.

In den extrazellulären Raum werden nur wenige Proteine aktiv exportiert. Zwar ist gerade bei höheren Zelldichten ein relativ hoher Proteinanteil im Medium zu finden, dies lässt sich aber meist auf ein Austreten aus dem Periplasma durch hohe Proteinkonzentrationen und erhöhter Porosität der äußeren Membran zurückführen, nicht aber auf ein aktives Sekretionssystem. Diese Porosität kann durch die Zugabe von Triton X-100 oder Glycin in das Kulturmedium noch verstärkt werden (Jang et al. 1999). Die Porosität der Zellwand kann ebenfalls durch die Koexpression von BRP verstärkt werden (van der Wal, FJ et al. (1998), Appl. Environ. Microbiol. 64). Dies kann jedoch auch zum vermehrten Austritt von anderen zellulären Proteinen und zu starker Zellschädigung führen. Eine andere Möglichkeit, um einen extrazellulären Transport zu verstärken, ist die Fusion mit Proteinen der äußeren Membran, z.B. OmpF.

Ein Peptid, das einen effizienten Transport von rekombinanten Proteinen durch die Cytoplasmamembran ermöglicht, ist ein nützliches Werkzeug in der Biotechnologie. Gerade die Sekretion großer komplexer Proteine bereitet oft Probleme. Alternativen zu den schon bekannten Strategien zur Sekretion sind daher wünschenswert.

Aufgabe der Erfindung ist daher, ein neuartiges Sekretionssystem zur Verfügung zu stellen, das den Transport von in *E*. *coli*-Zellen rekombinant exprimierten Proteinen in das Periplasma bzw. in das Kulturmedium ermöglicht.

Erfindungsgemäß wird die Aufgabe gelöst durch die Verwendung einer Peptidsequenz gemäß einer der SEQ-ID No. 2, 4, 6 oder 8 oder einer Teilsequenz davon, oder einer zu mindestens einer dieser Sequenzen homologen Peptidsequenz mit mindestens 70 % Sequenzidentität, bevorzugt 80 % Sequenzidentität, besonders bevorzugt 90 % Sequenzidentität, als Sekretionssignal, das den Transport von Proteinen, insbesondere hochmolekularen Proteinen über die Cytoplasmamembran von *E*. *coli* vermittelt. Die erfindungsgemäß verwendete Protein- bzw. Peptidsequenz vermittelt dabei die Sekretion von Proteinen aus der Bakterienzelle in das Periplasma und/oder in den extrazellulären Raum.

Bei den erfindungsgemäß verwendeten Peptidsequenzen handelt es sich um die Sequenzen von S-Layer-Proteinen oder Teilsequenzen davon, oder um eine zu mindestens einer dieser Sequenzen homologe Sequenz mit mindestens 70 % Sequenzidentität, bevorzugt 80 % Sequenzidentität, besonders bevorzugt 90 % Sequenzidentität.

Wird eine Teilsequenz der Peptidsequenzen verwendet, so besitzt diese eine Länge von mindestens 100 Aminosäuren, vorzugsweise mindestens 150 Aminosäuren, bevorzugt mindestens 200 Aminosäuren, besonders bevorzugt mindestens 250 Aminosäuren.

Bei den erfindungsgemäß verwendeten S-Layer-Proteinen handelt es sich um stumme und/oder funktionale S-Layer-Proteine der Bakteriengattungen *Lysinibacillus,* Teilsequenzen davon, oder zu mindestens einer dieser Sequenzen homologe Peptidsequenzen mit mindestens 70 % Sequenzidentität, bevorzugt 80 % Sequenzidentität, besonders bevorzugt 90 % Sequenzidentität. Bevorzugt stammen die S-Layer-Proteine von den Spezies *Lysinibacillus sphaericus,* besonders bevorzugt von den Stämmen *Lysinibacillus sphaericus* JG-A12 (Isolat einer Uranabraumhalde) (Genbank-Akzessionsnr. AJ866975) und/oder *Lysinibacillus sphaericus* NCTC 9602 (Genbank-Akzessionsnr. AJ866974).

Ganz besonders bevorzugt handelt es sich bei den S-Layer-Proteinen um die S-Layer-Proteine SlfA (Genbank-Akzessionsnr. AJ849547; **SEQ ID-No.** 2), SllA (Genbank-Akzessionsnr. AJ849548; **SEQ ID-No. 4**), SlfB (Genbank-Akzessionsnr. AJ849549; **SEQ ID-No. 6)** und/oder SIIB (Genbank-Akzessionsnr. AJ849550; **SEQ ID-No. 8)** von *Lysinibacillus sphaericus* JG-A12.

Werden diese Proteine oder Teilsequenzen davon in *E. coli* Zellen exprimiert, werden sie aus dem Cytosol zunächst in das Periplasma sezerniert. Ebenso werden rekombinante Fusionsproteine, die aus einem zu exprimierenden Protein (Zielprotein) und einer erfindungsgemäß verwendeten Peptidsequenz bestehen, durch die äußere Membran in das Nährmedium transportiert. Die erfindungsgemäß verwendeten Peptidsequenzen eignen sich daher als Sekretionssignal, welches an ein Zielprotein fusioniert wird und so den Transport des Fusionsproteins aus Zielprotein und Sekretionssignal in das Periplasma bzw. das Nährmedium vermittelt.

Dies ist überraschend, da diese Sequenzen von einem gram-positiven Bakterium stammen. Gram-positive Bakterien wie *Lysinibacillus* haben eine andere Membranstruktur und andere Transportsysteme als gram-negative Bakterien. Es ist daher überraschend, dass aus einem gram-positiven Bakterium stammende Sequenzen als Sekretionssignale in gram-negativen Bakterien funktionieren.

Für den Transport der Proteine aus dem Cytosol in das Periplasma und/oder in den extrazellulären Raum ist insbesondere derjenige Sequenzbereich, der sich von 824 Aminosäuren bis zum C-Terminus des entsprechenden S-Layer-Proteins erstreckt, entscheidend. Wird also nur eine Teilsequenz der Sequenzen **SEQ ID-No. 2, 4, 6,** oder **8** als Sekretionssignal verwendet, umfasst diese bevorzugt diesen C-terminalen Abschnitt einer der erfindungsgemäß verwendeten Sequenzen oder eine Teilsequenz dieses C-terminalen Abschnitts mit einer Länge von mindestens 100 Aminosäuren, vorzugsweise mindestens 150 Aminosäuren, bevorzugt mindestens 200 Aminosäuren, besonders bevorzugt mindestens 250 Aminosäuren.

Als Sekretionssignal ist der Bereich besonders bevorzugt, der dem Abschnitt von 824 bis 1101 Aminosäuren des S-Layer-Proteins SllB (Genbank-Akzessionsnr. AJ849550) von *Lysinibacillus sphaericus* entspricht (**SEQ ID-No. 9**).

Die erfindungsgemäß verwendete Peptidsequenz umfasst also bevorzugt mindestens eine Sequenz der **SEQ ID-No. 9,** eine Teilsequenz davon mit einer Länge von mindestens 100 Aminosäuren, vorzugsweise mindestens 150 Aminosäuren, bevorzugt mindestens 200 Aminosäuren, besonders bevorzugt mindestens 250 Aminosäuren, oder eine zu einer dieser Peptidsequenzen homologe Sequenz mit mindestens 50 % Sequenzidentität, vorzugsweise 70 % Sequenzidentität, bevorzugt 80 % Sequenzidentität, besonders bevorzugt 90 % Sequenzidentität.

Das erfindungsgemäße Sekretionssignal ist die Grundlage eines neuen, bislang unbekannten Systems zur Sekretion von rekombinanten Proteinen aus *E*. *coli.* Verwendung findet dieses System in der Biotechnologie und Molekularbiologie. Die für ein Sekretionssignal kodierende Nukleinsäuresequenz wird vorzugsweise durch molekularbiologische Methoden mit einer für ein zu exprimierendes Protein (Zielprotein) kodierenden Nukleinsäuresequenz fusioniert. Das daraus entstehende Fusionsprotein eignet sich als Vehikel für den Transport des Zielproteins durch die Cytoplasmamembran von *E*. *coli*. Die Fusionsproteine werden nach der Expression durch die Sekretionsmaschinerie der *E. coli*-Zelle in das Periplasma, aber auch in das die *E*. *coli*-Zelle umgebende Kulturmedium transportiert. Insbesondere für die Sekretion von rekombinanten Proteinen mit hohem Molekulargewicht ist dieses System hervorragend geeignet.

Gegenstand der Erfindung ist auch die Verwendung einer Nukleinsäuresequenz, die für ein erfindungsgemäßes Sekretionssignal kodiert, zur Expression eines Sekretionssignals in *E*. *coli*-Zellen, wobei das Sekretionssignal den Transport von Proteinen aus dem Cytosol der *E*. *coli*-Zelle in das Periplasma und/oder den extrazellulären Raum vermittelt.

Bei der Nukleinsäuresequenz handelt es sich bevorzugt um die kodierende Sequenz der S-Layer-Gene *slfA* (**SEQ ID-Nr**. **1**), *sllA* (**SEQ ID-Nr**. **3**), *slfB* (**SEQ ID-Nr**. **5**) und *sll*B (**SEQ ID-Nr. 7)** oder eine dazu homologe Nukleinsäuresequenz mit mindestens 50 % Sequenzidentität, vorzugsweise 70 % Sequenzidentität, bevorzugt 80 % Sequenzidentität, besonders bevorzugt 90 % Sequenzidentität, oder eine Teilsequenz davon mit einer Länge von mindestens 300 Basenpaaren, vorzugsweise mindestens 450 Basenpaaren, bevorzugt mindestens 600 Basenpaaren, besonders bevorzugt mindestens 750 Basenpaaren. Bevorzugt werden als Teilsequenzen diejenigen Sequenzbereiche verwendet, die sich von 2473 bp bis zum Stoppcodon (Ende des offenen Leserahmens) erstrecken, oder eine Teilsequenz davon mit einer Länge von mindestens 300 Basenpaaren, vorzugsweise mindestens 450 Basenpaaren, bevorzugt mindestens 600 Basenpaaren, besonders bevorzugt mindestens 750 Basenpaaren.

Besonders bevorzugt als erfindungsgemäßes Sekretionssignal ist der Sequenzabschnitt von 2473 bis 3303 bp des *sll*B-Gens (Genbank-Akzessionsnr. AJ849550; **SEQ ID-No**. **10**) oder eine Teilsequenz davon mit einer Länge von mindestens 300 Basenpaaren, vorzugsweise mindestens 450 Basenpaaren, bevorzugt mindestens 600 Basenpaaren, besonders bevorzugt mindestens 750 Basenpaaren.

Erfindungsgemäß wird das Sekretionssignal zur Herstellung eines Fusionsproteins verwendet, welches ein zu exprimierendes Protein (Zielprotein) und das Sekretionssignal umfasst.

Gegenstand der Erfindung ist daher auch die Verwendung einer Nukleinsäuresequenz, welche für ein solches Fusionsprotein kodiert. Dabei ist die kodierende Sequenz des zu exprimierenden Zielproteins bzw. ggf. eines Zielproteinfragments an die für das Sekretionssignal kodierende Sequenz im gleichen Leseraster C-terminal oder N-terminal fusioniert. Das Fusionsprotein aus Sekretionssignal und Zielprotein wird nach der Expression in der Wirtszelle über die Cytoplasmamembran in den periplasmatischen Raum und/oder in das die Wirtszelle umgebende Medium transportiert.

Um das Fusionsprotein zu erhalten, muss die für dieses Fusionsprotein kodierende Nukleinsäuresequenz in einer Wirtszelle exprimiert werden. Gegenstand der Erfindung ist daher auch die Verwendung einer Expressionskassette oder eines Expressionsvektors, der eine für ein erfindungsgemäß hergestelltes Fusionsprotein kodierende Nukleinsäure umfasst.

Eine Klonierungskassette oder eines Klonierungsvektors zur Herstellung der erfindungsgemäß verwendeten Expressionskassette oder eines Expressionsvektors umfasst eine Klonierungsstelle für die Insertion des Zielproteins und die für das Sekretionssignal kodierende Nukleinsäuresequenz. Diese Klonierungskassette bzw. -vektor erlaubt vorteilhaft die Insertion einer für das Zielprotein kodierenden Nukleinsäuresequenz entweder N-terminal oder C-terminal im gleichen Leseraster wie die für das Sekretionssignal kodierende Sequenz. Auf diese Weise kann jedes gewünschte Zielprotein mit dem Sekretionssignal zu einem Fusionsprotein fusioniert und in eine Wirtszelle transformiert und exprimiert werden.

Oft soll das Zielprotein nach der Expression wieder von dem Sekretionssignal abgetrennt werden. Bevorzugt enthält die Expressionskassette oder die Klonierungskassette eine für eine Proteaseschnittstelle kodierende Nukleinsäuresequenz, durch den das Zielprotein nach Expression mittels einer Protease vom Sekretionssignal getrennt werden kann. Der Begriff "Protease" umfasst dabei alle gebräuchlichen Endo- und Exoproteinasen, wobei Endoproteinasen bevorzugt sind. Aus dem Stand der Technik sind zahlreiche Schnittstellen für Proteasen, die für solche Zwecke, d. h. das Abtrennen von Fusionsanteilen bei der Expression rekombinanter Fusionsproteine, einsetzbar sind, bekannt, wie z. B. die sauren, neutralen und alkalischen Proteasen aus den Gruppen der Serin-, Cystein-, Aspartat- oder Metallproteasen. Bevorzugt wird als Proteaseschnittstelle die Schnittstelle der natürlicherweise in humanem Blut vorkommenden Faktor-Xa-Protease, die für die Aktivierung von Prothrombin zu Thrombin verantwortlich ist.

Über einen geeigneten Promotor wird die Expression des Fusionsproteins in der Wirtszelle bewirkt. Zu diesem Zweck steht der Promotor unter der Kontrolle eines regulatorischen Gens, das entweder im Genom der Wirtszelle oder auf dem Expressionsvektor vorhanden ist. Dabei erfolgt die Expression entweder konstitutiv oder bevorzugt induzierbar. Die Expression des Fusionsproteins wird bevorzugt dann gezielt induziert, wenn in der Kultur eine bestimmte Zahl an Wirtszellen erreicht ist.

Diese Induktion erfolgt beispielsweise im Fall der *lac-, Ipp-,* und darauf basierenden Hybridpromotoren durch die Gabe von IPTG, im Fall des *trp*-Promotors durch Tryptophan-Mangel oder die Gabe von Indolacrylsäure, im Fall des *araBAD*-Promotors durch die Gabe von Arabinose, im Fall des *tetA*-Promotors durch die Gabe von Tetracyclin oder im Fall des *nar*-Promotors durch die Gabe von Nitrat-lonen. Alternativ wird die Expression auch thermisch, d.h. durch Temperaturveränderung, induziert, indem die verwendeten Promotoren unter die Kontrolle von temperatursensitiven Repressoren gestellt werden, die bei Temperaturänderung die Promotoraktivität nicht mehr reprimieren können.

Für die Expression des Fusionsproteins in einer prokaryotischen Wirtszelle enthält der Expressionsvektor bevorzugt außerdem 6 bis 7 Nukleotide stromaufwärts von dem für das Fusionsprotein kodierenden Nukleinsäureabschnitt eine Shine-Dalgarno-Sequenz, die zusammen mit der kodierenden Sequenz transkribiert wird und in vielen prokaryotischen Wirtszellen den Startpunkt für die Translation durch das Ribosom bildet. Bevorzugt enthält der Expressionsvektor außerdem stromabwärts von dem das Fusionsprotein kodierenden Nukleinsäureabschnitt einen Transkriptionsterminator, der gleichzeitig den Endpunkt der Transkription darstellt und in der transkribierten mRNA Sekundärstrukturen ausbildet, die die Degradation der mRNA verzögern und ihre Halbwertszeit verlängern. Weitere bevorzugte Bestandteile des Expressionsvektors sind ein oder mehrere Gene, die der Wirtszelle die Resistenz gegen ein oder mehrere Antibiotikum vermitteln und so die Selektion von Wirtszellen ermöglichen, die den Expressionsvektor aufgenommen haben, und eine ori-Sequenz (*origin of replication*; Replikationsursprung), durch welche die Replikation des Expressionsvektors durch die Replikationsmaschinerie der Wirtszelle ermöglicht wird.

Gegenstand der Erfindung ist auch die Verwendung einer *E*. *coli*-Wirtszelle, die eine erfindungsgemäß verwendete Expressionskassette oder einen Expressionsvektor umfasst. Der Begriff Wirtszelle umfasst transient (d. h. vorübergehend) transfizierte Zellen (z. B. durch mRNA), plasmidtransformierte Wirtszellen wie auch Wirtszellen, in denen die erfindungsgemäße Expressionskassette oder der Expressionsvektor stabil in das Genom integriert ist. Gegenstand der Erfindung ist weiterhin die Verwendung eines Kits, welcher folgende Bestandteile umfasst
a. eine Klonierungskassette oder Klonierungsvektor umfassend:
   - eine für ein erfindungsgemäßes Sekretionssignal kodierende Nukleinsäuresequenz und
   - eine Klonierungsstelle, in welche die für das Zielprotein kodierende Nukleinsäuresequenz einkloniert werden kann,
      wobei die für das Zielprotein kodierende Nukleinsäuresequenz an die für das Sekretionssignal kodierende Sequenz im gleichen Leseraster C-terminal oder N-terminal fusioniert ist,
   oder eine erfindungsgemäß verwendete Expressionskassette oder Expressionsvektor
b. kompetente *E*. *coli*-Zellen und ggf. Kontrollreagenzien, Puffer oder Zelltransformationsreagenzien,
zur Expression eines erfindungsgemäßen Fusionsproteins *in E*. *coli*, wobei das Sekretionssignal den Transport von Proteinen aus dem Cytosol der *E*. *coli*-Zelle in das Periplasma und/oder den extrazellulären Raum vermittelt.

Gegenstand der Erfindung ist außerdem ein Verfahren zur Expression und Sekretion eines Fusionsproteins aus *E*. *coli*-Wirtszellen, welches die folgenden Schritte umfasst:
a) Transformation von *E*. *coli*-Zellen mit einer erfindungsgemäß verwendeten Expressionskassette oder einem Expressionsvektor und
b) Induktion der Expression des Fusionsproteins, wobei das Sekretionssignal den Transport von Proteinen aus dem Cytosol der *E*. *coli*-Zelle in das Periplasma und/oder den extrazellulären Raum vermittelt.

Besonders bevorzugt ist die Verwendung des Sekretionssignals für die Expression und Sekretion von S-Layer-Proteinen.

Eine weitere Anwendungsmöglichkeit betrifft die Verwendung der Erfindung zur Expression von rekombinanten Proteinen an der Oberfläche bakterieller Zellen (heterologous surface display, bacterial surface display). Dabei werden die S-Layer Proteine als Vehikel für andere Proteine (z. B. Peptide, Enzyme) zur Sekretion, aber auch zur Anheftung an die Zelloberfläche genommen. N-terminale Sequenzbereiche des S-Layer-Proteins heften sich an Membranstrukturen an, so auch an die äußere Membran von *E. coli.* Derartige Systeme eignen sich z. B. zur Entwicklung von Impfstoffen, zur Entwicklung von Biokatalysatoren und zur Entwicklung spezifischer Bindestrukturen.

Bevorzugt werden Teilsequenzen des S-Layer-Proteins so ausgewählt, dass die Fusionsproteine unter bestimmten definierten Bedingungen aggregieren und makromolekulare Strukturen ausbilden. Dies ermöglicht vorteilhaft eine kostengünstige und unaufwändige Aufreinigung der Fusionsproteine durch Zentrifugation oder durch Ausfällung.

Vorteilhaft wachsen die die Fusionsproteine exprimierenden *E*. *coli*-Zellen nach Induktion der Expression unter Standard-Wachstumsbedingungen, wie sie in Standardwerken wie z. B. Sambrook et al. (1989) beschrieben werden, zu höheren Zelldichten heran (bis zu A600 = 5,0), als dies bei entsprechenden nicht-transformierten *E*. *coli*-Zellen der Fall ist. Damit eignen sie sich zur effizienten Produktion von rekombinanten Proteinen. Als Wachstumsbedingungen wird dabei eine geeignete Menge LB-Medium, bevorzugt zwischen 50 bis 1000 ml, in einen Erlenmeyer-Schüttelkolben, der etwa das fünffache Volumen fasst, gefüllt und Schüttelfrequenzen zwischen 200 bis 300 Umdrehungen/min, vorzugsweise von 220 bis 250 Umdrehungen/min gewählt.

Die Produktion der Fusionsproteine durch die *E*. *coli*-Zellen erfolgt vorzugsweise bei Temperaturen unter 37 °C, bevorzugt bei weniger als 25 °C, besonders bevorzugt zwischen 10 und 14 °C.

Die folgenden Ausführungsbeispiele erläutern die Erfindung näher, ohne sie auf diese zu beschränken.

### Ausführungsbeispiel 1: Expression und Sekretion von GFP vermittelt durch das erfindungsgemäße Sekretionssignal

Als Beispiel wird N-terminal verkürztes SIIB mit dem Grün fluoreszierenden Protein (green fluorescent protein; GFP) fusioniert. Zunächst wird das stumme S-Layer Gen *sll*B (Accessionsnummer AJ849550) des Stammes *Lysinibacillus sphaericus* JG-A12 (Accessionsnummer AJ866975) ohne Signalpeptid und N-terminaler Domäne unter Verwendung von genfragmentspezifischen Primern Lic704f mit der **SEQ ID-No. 11** [5'gacgacgacaagatgatcaacaacacaactgttgaa'3] und Lic_PI mit der **SEQ ID-No. 12** [5'gaggagaagcccggttggagttggctttactgtaata'3] durch PCR vervielfältigt. Das erhaltene PCR-Produkt (Länge 2599 bp) wird in den Vektor pET-30 Ek/Lic (Novagen) ligiert. Der Vektor mit dem *sll*B-Insert wird zunächst in *E*. *coli* NovaBlue (Novagen) transformiert. Von positiven Klonen werden Plasmide isoliert.

Anschließend wird das für das Grün fluoreszierende Protein (GFP) kodierende Gen gfp mit sequenzspezifischen Primern amplifiziert und über die Primer Schnittstellen für Restriktionsenzyme eingeführt. Das PCR-Produkt wird nach Standardprotokollen aufgereinigt und mit den entsprechenden Restriktionsenzymen geschnitten. Ebenso wird der Vektor mit *sll*B-Insert mit diesen Enzymen geschnitten. Eine Ligation von *gfp* erfolgt nach Standardprotokollen. Der Vektor mit dem fusionierten Gen wird zunächst in *E*. *coli* NovaBlue (Novagen) transformiert. Von positiven Klonen werden Plasmide isoliert, die in den Expressionsstamm *E. coli* BI21(DE3) transformiert werden.

Für die Expression von der für das SIIB-GFP-Fusionsprotein kodierenden DNA-Sequenz wird zunächst eine Vorkultur der transformierten *E. coli* BI21(DE3) in LB-Medium angezogen. Diese Vorkultur wird in frisches LB-Medium überimpft und bis zu einer O.D. von ca. 0,1 inkubiert. Das Wachstum erfolgt bei Raumtemperatur (< 25°C). Nach ca. 2 h Wachstum erfolgt eine Zugabe von 0,1 mM IPTG und damit eine Induktion der Expression der rekombinanten Fusionsproteine.

Die rekombinanten Fusionsproteine werden sowohl in den periplasmatischen Raum transportiert als auch in das Kulturmedium abgegeben. Die Fusionsproteine können daraus aufgereinigt werden.

### Ausführungsbeispiel 2: Aufreinigung der sezernierten Fusionsproteine

Das folgende Protokoll dient zur einfachen Gewinnung der periplasmatischen Fraktion. Das Prinzip beruht auf einem osmotischem Schock, dem die Zellen ausgesetzt werden.

Zunächst wird das Zellpellets in 1,5 ml 30 mM Tris-HCI, 20 % Saccharose (pH 8) und EDTA (Endvolumen 1mM) pH 8,0 resuspendiert und bei RT für 10 min langsam gerührt. Anschließend wird die Resuspension bei 10000 * g für 10 min bei 4 °C zentrifugiert und der Überstand verworfen. Die Pellets werden nun in 200 µl eiskaltem 5 mM MgSO₄ resuspendiert und für 10 min auf Eis langsam gerührt. Während dieses Schrittes wird die periplasmatische Fraktion in den Puffer entlassen. Die Zellen werden bei 10000 * g, 4 °C für 10 min abzentrifugiert, und der Überstand, der die Fusionsproteine enthält, in ein frisches Reaktionsgefäß überführt. Der Erfolg der Methode kann lichtmikroskopisch untersucht werden, indem man die Zellen vor und nach ihrer Behandlung vergleicht, denn vor der Behandlung sind die Zellen i.d. Regel stäbchenförmig, danach sind die Zellen durch das Aufbrechen der Zellwand und den Verlust des Turgors rund.

Zur Gewinnung der extrazellulären Proteine aus dem Nährmedium wird der Kulturüberstand nach Zellernte durch Filterzentrifugation mit Vivaspin 500 Säulen der Firma Sartorius aufkonzentriert. Alternativ werden die Proteine durch die Zugabe von Ammoniumsulfat ausgefällt. Der Niederschlag wird abzentrifugiert und gegen dest. H₂O in einem zweiten Schritt gegen eine 10 mM CaCl₂-Lösung dialysiert

## Patentansprüche

1. Verwendung einer S-Layer-Protein-Peptidsequenz als Sekretionssignal in *E*. *coli*-Zellen, wobei die Peptidsequenz ausgewählt ist aus den SEQ-ID No. 2, 4, 6 und 8 oder einer Teilsequenz davon mit mindestens 100 Aminosäuren oder einer zu mindestens einer dieser Sequenzen homologen Peptidsequenz mit mindestens 70 % Sequenzidentität und wobei das Sekretionssignal den Transport von Proteinen aus dem Cytosol der *E*. *coli-*Zelle in das Periplasma und/oder den extrazellulären Raum vermittelt.

2. Verwendung einer Nukleinsäuresequenz kodierend für ein Sekretionssignal wie in Anspruch 1 definiert zur Expression des Sekretionssignals in *E*. *coli*-Zellen, wobei das Sekretionssignal den Transport von Proteinen aus dem Cytosol der *E*. *coli*-Zelle in das Periplasma und/oder den extrazellulären Raum vermittelt.

3. Verwendung einer Nukleinsäuresequenz kodierend für ein Fusionsprotein umfassend ein zu exprimierendes Zielprotein und ein Sekretionssignal wie in Anspruch 1 definiert, wobei die für das Sekretionssignal kodierende Sequenz 5' oder 3' an die kodierende Sequenz des zu exprimierenden (Ziel)proteins oder -proteinfragments im gleichen Leseraster fusioniert ist. zur Expression des Fusionproteins in *E*. *coli,* wobei das Sekretionssignal den Transport von Proteinen aus dem Cytosol der *E*. *coli*-Zelle in das Periplasma und/oder den extrazellulären Raum vermittelt.

4. Verwendung einer Expressionskassette oder eines Expressionsvektor umfassend eine Nukleinsäure, wie in Anspruch 3 definiert, zur Expression eines Fusionsproteins in *E*. *coli,* wobei das Sekretionssignal den Transport von Proteinen aus dem Cytosol der *E*. *coli-*Zelle in das Periplasma und/oder den extrazellulären Raum vermittelt.

5. Verwendung einer *E*. *coli*-Wirtszelle umfassend eine Expressionskassette oder einen Expressionsvektor, wie in Anspruch 4 definiert, zur Expression eines Fusionproteins, wobei das Sekretionssignal den Transport von Proteinen aus dem Cytosol der *E*. *coli-*Zelle in das Periplasma und/oder den extrazellulären Raum vermittelt.

6. Verwendung eines Kits umfassend:
a. eine Klonierungskassette oder einen Klonierungsvektor umfassend:
- eine für ein Sekretionssignal kodierende Nukleinsäuresequenz wie in Anspruch 2 definiert und
- eine Klonierungsstelle, in welche die für das Zielprotein kodierende Nukleinsäuresequenz einkloniert werden kann,
wobei die für das Zielprotein kodierende Nukleinsäuresequenz an die für das wie in Anspruch 1 definierte Sekretionssignal kodierende Sequenz im gleichen Leseraster C-terminal oder N-terminal fusioniert ist,
oder eine Expressionskassette oder einen Expressionsvektor wie in Anspruch 4 definiert,
b. kompetente *E*. *coli*-Zellen und ggf. Kontrollreagenzien, Puffer oder Zelltransfektionsreagenzien,
zur Expression eines Fusionsproteins *in E. coli,* wobei das Sekretionssignal den Transport von Proteinen aus dem Cytosol der *E. coli*-Zelle in das Periplasma und/oder den extrazellulären Raum vermittelt.

7. Verfahren zur Expression und Sekretion eines Fusionsproteins aus *E*. *coli*-Wirtszellen, umfassend die Schritte.
a. Transformation von *E*. *coli*-Zellen mit einer Expressionskassette oder einem Expressionsvektor wie in Anspruch 4 definiert und
b. Induktion der Expression des Fusionsproteins, wobei das Sekretionssignal den Transport von Proteinen aus dem Cytosol der *E*. *coli*-Zelle in das Periplasma und/oder den extrazellulären Raum vermittelt.

## Claims

1. Use of an S-layer protein peptide sequence as a secretion signal in *E*. *coli* cells, wherein the peptide sequence is selected from the SEQ-ID No. 2, 4, 6 and 8 or a partial sequence thereof comprising at least 100 amino acids or a peptide sequence which is homologous to at least one of these sequences and has at least 70 % sequence identity and wherein the secretion signal facilitates the transport of proteins from the cytosol of the *E*. *coli* cell into the periplasm and/or the extracellular space.

2. Use of a nucleic acid sequence coding for a secretion signal according to claim 1 for expressing the secretion signal in *E*. *coli* cells, wherein the secretion signal facilitates the transport of proteins from the cytosol of the *E*. *coli* cell into the periplasm and/or the extracellular space.

3. Use of a nucleic acid sequence coding for a fusion protein comprising a target protein to be expressed and a secretion signal according to claim 1, wherein the sequence 5' or 3' coding for the secretion signal is fused to the coding sequence of the (target) protein or (target) protein fragment to be expressed in the same reading frame in order to express the fusion protein in *E*. *coli*, wherein the secretion signal facilitates the transport of proteins from the cytosol of the *E*. *coli* cell into the periplasm and/or the extracellular space.

4. Use of an expression cassette or an expression vector comprising a nucleic acid, according to claim 3, for expressing a fusion protein in *E*. *coli*, wherein the secretion signal facilitates the transport of proteins from the cytosol of the *E*. *coli* cell into the periplasm and/or the extracellular space.

5. Use of an *E*. *coli* host cell comprising an expression cassette or an expression vector, according to claim 4, for expressing a fusion protein, wherein the secretion signal facilitates the transport of proteins from the cytosol of the *E*. *coli* cell into the periplasm and/or the extracellular space.

6. Use of a kit comprising:
a. a cloning cassette or a cloning vector including:
- a nucleic acid sequence, coding for a secretion signal, according to claim 2 and
- a cloning site into which the nucleic acid sequence coding for the target protein can be cloned,
wherein the nucleic acid sequence coding for the target protein is fused to the sequence coding for the secretion signal according to claim 1 in the same reading frame at the C-terminal or N-terminal,
or an expression cassette or an expression vector according to claim 4,
b. competent *E*. *coli* cells and optionally control reagents, buffers or cell transfection reagents,
for expressing a fusion protein in *E*. *coli,* wherein the secretion signal facilitates the transport of proteins from the cytosol of the *E*. *coli* cells into the periplasm and/or the extracellular space.

7. Process for expressing and secreting a fusion protein from *E*. *coli* host cells, comprising the steps of:
a. transforming *E*. *coli* cells using an expression cassette or an expression vector according to claim 4 and
b. inducing the expression of the fusion protein, wherein the secretion signal facilitates the transport of proteins from the cytosol of the *E. coli* cells into the periplasm and/or the extracellular space.

## Revendications

1. Utilisation d'une séquence peptidique de protéines S-Layer en tant que signal de sécrétion dans des cellules d'*E*. *coli*, dans laquelle la séquence peptidique est sélectionnée parmi les SEQ-ID n°2, 4, 6 et 8 ou une séquence partielle de ceux-ci avec au moins 100 acides aminés ou une séquence peptidique homologue à au moins une de ces séquences avec au moins 70 % d'identité de séquence, et dans laquelle le signal de sécrétion assure le transport de protéines à partir du cytosol de la cellule d'*E*. *coli* dans le périplasme et/ou dans l'espace extracellulaire.

2. Utilisation d'une séquence d'acide nucléique codant pour un signal de sécrétion selon la définition de la revendication 1, pour l'expression du signal de sécrétion dans des cellules d'*E*. *coli,* dans laquelle le signal de sécrétion assure le transport de protéines à partir du cytosol de la cellule d'*E*. *coli* dans le périplasme et/ou dans l'espace extracellulaire.

3. Utilisation d'une séquence d'acide nucléique codant pour une protéine de fusion comprenant une protéine cible à exprimer et un signal de sécrétion selon la définition de la revendication 1, dans laquelle la séquence 5' ou 3' codant pour le signal de sécrétion est fusionnée à la séquence codante de la protéine (cible) ou du fragment de protéine (cible) à exprimer dans le même cadre de lecture pour l'expression de la protéine de fusion dans *E*. *coli*, dans laquelle le signal de sécrétion assure le transport de protéines à partir du cytosol de la cellule d'*E*. *coli* dans le périplasme et/ou dans l'espace extracellulaire.

4. Utilisation d'une cassette d'expression ou d'un vecteur d'expression comprenant un acide nucléique selon la définition de la revendication 3, pour l'expression d'une protéine de fusion dans *E*. *coli,* dans laquelle le signal de sécrétion assure le transport de protéines à partir du cytosol de la cellule d'*E*. *coli* dans le périplasme et/ou dans l'espace extracellulaire.

5. Utilisation d'une cellule hôte d'*E*. *coli*, comprenant une cassette d'expression ou un vecteur d'expression, selon la définition de la revendication 4, pour l'expression d'une protéine de fusion, dans laquelle le signal de sécrétion assure le transport de protéines à partir du cytosol de la cellule d'*E*. *coli* dans le périplasme et/ou dans l'espace extracellulaire.

6. Utilisation d'un kit comprenant :
a. une cassette de clonage ou un vecteur de clonage comprenant :
- une séquence d'acide nucléique codant pour un signal de sécrétion selon la définition de la revendication 2, et
- un site de clonage dans lequel la séquence d'acide nucléique codant pour la protéine cible peut être clonée,
dans laquelle la séquence d'acide nucléique codant pour la protéine cible est fusionnée, dans le même cadre de lecture, au niveau de l'extrémité C-terminale ou N-terminale, à la séquence codante pour le signal de sécrétion selon la définition de la revendication 1,
ou une cassette d'expression ou un vecteur d'expression selon la définition de la revendication 4,
b. des cellules d'*E*. *coli* compétentes et éventuellement des réactifs de contrôle, des tampons ou des réactifs de transfection des cellules,
pour l'expression d'une protéine de fusion dans *E*. *coli,* dans laquelle le signal de sécrétion assure le transport de protéines à partir du cytosol de la cellule d'*E*. *coli* dans le périplasme et/ou dans l'espace extracellulaire.

7. Procédé d'expression et de sécrétion d'une protéine de fusion à partir de cellules hôtes d'*E*. *coli*, comprenant les étapes suivantes :
a. transformation de cellules d'*E*. *coli*, avec une cassette d'expression ou un vecteur d'expression selon la définition de la revendication 4, et
b. induction de l'expression de la protéine de fusion, dans laquelle le signal de sécrétion assure le transport de protéines à partir du cytosol de la cellule d'*E*. *coli* dans le périplasme et/ou dans l'espace extracellulaire.
